# EUROPEAN PATENT APPLICATION

(11) **EP 4 265 206 A1**
(43) Date of publication of application: **25.10.2023**
(21) Application number: 22382364.2
(22) Date of filing: 19.04.2022
(51) Int. Cl.: A61B 17/435

(54) **SYSTEM FOR EMBRYO TRANSFER**

(71) Applicant: Premium Fertility S.L, 46012 Valencia (ES)
(72) Inventor: SANTAMARIA COSTA, Xavier, 46012 Valencia (ES); SIMON VALLÉS, Carlos, 46012 Valencia (ES)
(74) Representative: Hoffmann Eitle

(57) **Abstract**

Disclosed are apparatuses for delivering a fertilized egg or embryo into a maternal uterine endometrium in humans or any other mammalian species. In one embodiment the system comprises a body (160) having a distal end (162) suitable for penetrating the endometrial epithelium; a plunger (165) slidably received in a lumen of the body (160); an actuator (195) operable advance the plunger (165) to expel a fertilized egg from the lumen of the body (160); a first injectable medium (111); and a second injectable medium (112) for containing the fertilized egg; wherein the first injectable medium (111) has a higher viscosity than the second injectable medium (112).

## Description

### TECHNICAL FIELD

The present disclosure relates to systems and methods for transferring a fertilized egg or embryo to the endometrial epithelium. The disclosure also relates to a device for guiding such systems and for engaging a speculum.

### BACKGROUND

Human In Vitro Fertilization (IVF) and Embryo Transfer (ET), first successfully performed in 1978, has become a widely practiced procedure to treat infertile couples who have failed with more conventional methods of therapy such as superovulation and intrauterine insemination. The most common indications for IVF and related procedures, such as Gamete In Vitro Fertilization or Gamete Intra-Fallopian Transfer (GIFT) which includes women having blocked or damaged fallopian tubes and includes low sperm and/or egg quality. Related factors include age of the female, and the degree of endometrial receptivity. The procedure may also be used in cases of severe male factor where direct (intracytoplasmic) injection of sperm is an option.

The IVF/ET procedure typically involves the hormonal stimulation of the female to first suppress her ability to ovulate on her own, then stimulate development of follicles in the ovaries with a fertility medication. The mature eggs are removed from the ovary transvaginally using a needle, preferably guided under ultrasound. Following harvesting of the eggs, the eggs are identified and sorted with regard to maturity, and then placed with a sperm sample from the male. Approximately 24 hours after fertilization, the eggs are examined to confirm fertilization, which occurs in approximately 65% to 85% of the eggs harvested.

After a short development period, the embryos are transferred, along with a volume of fluid, to the uterus using a delivery catheter. The delivery catheter is usually made of a soft plastic material to avoid damage to the endometrium. There are many potential difficulties in achieving a successful implantation. Because of the soft nature of the standard delivery catheter, in a number of cases, the tip of the catheter may bend back on itself or curve away from the fundus of the uterus. The tip may also accidentally pass between the layer of the endometrium and myometrium. Conversely, a stiffer catheter increases the risk of trauma to the uterus or cervix, with the latter possibly leading to the release of prostaglandins and expulsion of the eggs from the endometrium.

One particular difficulty in achieving successful implantation is the difficulty the surgeon has in visualizing the uterus and the endometrium into which the embryo is implanted. In particular, mucus and other bodily fluids may make it difficult for the surgeon to determine whether the endometrial epithelium has been reached.

It has also been observed that the fertilized egg can be expelled from the endometrial epithelium or dislodged after the transfer procedure, increasing the risk of a failed procedure. Similarly, the fertilized egg may remain stuck to the transfer apparatus even after the medium containing the fertilized egg is injected into the endometrial epithelium.

Further, a wide variety of speculums are used of different shapes and sizes, making it difficult for an embryo transfer apparatus to spatially lock to the anatomy of the patient in order to increase the accuracy of the embryo delivery.

There is therefore a need for improved embryo transfer apparatuses with an increased reliability and for a way of locking such embryo transfer apparatuses to a variety of speculum shapes.

### SUMMARY OF THE INVENTION

According to a first aspect of the invention, there is provided a system for delivering a fertilized egg or embryo into a maternal uterine endometrium in humans or any other mammalian species, comprising: a body having a distal end suitable for penetrating the endometrial epithelium; a plunger slidably received in a lumen of the body; an actuator operable advance the plunger to expel a fertilized egg from the lumen of the body; a first injectable medium; and a second injectable medium for containing the fertilized egg; wherein the first injectable medium has a higher viscosity than the second injectable medium.

According to a second aspect of the invention, there is provided a method of loading a system for delivering a fertilized egg or embryo into a maternal uterine endometrium in humans or any other mammalian species, the system comprising: a body having a distal end suitable for penetrating the endometrial epithelium; a plunger slidably received in a lumen of the body; and an actuator operable to advance the plunger to expel a fertilized egg from the lumen of the body; wherein the method comprises: drawing a first injectable medium into the lumen; and subsequently drawing a second injectable medium containing a fertilized egg into the lumen; wherein the first injectable medium has a higher viscosity than the second injectable medium.

According to a third aspect of the invention, there is provided an apparatus suitable for delivering a fertilized egg or embryo into a maternal uterine endometrium in humans or any other mammalian species, comprising a body configured to fit within a lumen of the female reproductive system, the body comprising: one or more lumens extending from a proximal end of the body to a distal portion of the body and having a distal opening at the distal portion of the body, wherein a first lumen of the one or more lumens slidably receives an inner body having a distal end suitable for penetrating the endometrial epithelium, the inner body comprising an inner lumen for receiving a fertilized egg; an actuator operable to expel the fertilized egg from the inner lumen; and a covering layer at least partially covering the distal opening of at least one lumen of the one or more lumens.

According to a fourth aspect of the invention, there is provided a device for engaging a speculum, comprising: two or more expandable engaging elements for outwardly engaging a speculum to fix the device to the speculum, wherein the two or more expandable engaging elements comprise a plurality of expanded configurations for engaging a plurality of speculum sizes; and a guide for guiding an embryo delivery device between the expandable engaging elements and through the speculum when the device is fixed to the speculum.

According to a fifth aspect of the invention, there is provided an apparatus suitable for delivering a fertilized egg or embryo into a maternal uterine endometrium in humans or any other mammalian species, comprising a body configured to fit within a lumen of the female reproductive system, the body comprising: a lumen extending from a proximal end of the body to a distal portion of the body and having a distal opening at the distal portion of the body, the lumen slidably receiving an inner body having a distal end suitable for penetrating the endometrial epithelium; the apparatus further comprising: a first actuator operable to advance the inner body out from the distal opening of the body; and a second actuator operable to expel a fertilized egg from an inner lumen of the inner body; wherein the inner body comprises a hydrophobic coating formed on a distal portion of the inner body.

### BRIEF DESCRIPTION OF THE DRAWINGS

To enable better understanding of the present disclosure, and to show how the same may be carried into effect, reference will now be made, by way of example only, to the accompanying schematic drawings, in which:
Fig. 1A shows a perspective view of an apparatus suitable for delivering a fertilized egg or embryo into a maternal uterine endometrium according to one or more embodiments shown and described herein.
Fig. 1B shows a perspective view of another apparatus suitable for delivering a fertilized egg or embryo into a maternal uterine endometrium according to one or more embodiments shown and described herein.
Fig. 1C shows a perspective view of another apparatus suitable for delivering a fertilized egg or embryo into a maternal uterine endometrium according to one or more embodiments shown and described herein.
Fig. 1D shows a side view of a distal portion of the apparatus shown in Fig. 1C.
Fig. 1E shows a front view of the apparatus shown in Fig. 1C.
Fig. IF shows a perspective view of another apparatus suitable for delivering a fertilized egg or embryo into a maternal uterine endometrium according to one or more embodiments shown and described herein.
Fig. 1G shows a perspective view of another apparatus suitable for delivering a fertilized egg or embryo into a maternal uterine endometrium according to one or more embodiments shown and described herein.
Fig. 1H shows a perspective view of another apparatus suitable for delivering a fertilized egg or embryo into a maternal uterine endometrium according to one or more embodiments shown and described herein.
Fig. 2A shows a schematic perspective view of a system for delivering a fertilized egg or embryo into a maternal uterine endometrium in a first configuration according to one or more embodiments shown and described herein.
Figs. 2B to 2D show schematic perspective views of the system of Fig. 2A in second to fourth configurations.
Fig. 2E shows a schematic view of a first, second and third medium situated in the endometrial epithelium.
Fig. 3 shows a schematic view of a bevelled tip according to one or more according to one or more embodiments shown and described herein.
Fig. 4 shows a diagram of an indicating device according to one or more embodiments shown and described herein.
Fig. 5 shows a diagram of a computer that may be used to control the apparatus according to one or more embodiments shown and described herein.
Fig. 6A shows a cross-sectional perspective view of a connector according to one or more embodiments shown and described herein.
Fig. 6B shows a close-up view of a part of the connector shown in Fig. 6A.
Fig. 6C shows a perspective view of a proximal end of a connector according to one or more embodiments shown and described herein.
Fig. 6D shows a perspective view of a distal end of a motion controller according to one or more embodiments shown and described herein.
Fig. 6E shows an exploded view of the connector shown in Fig. 6A.
Fig. 6F shows a perspective view of a connection between a body connector and a distal end of a motion controller according to one or more embodiments shown and described herein.
Fig. 6G shows a perspective view of a proximal portion of a motion controller according to one or more embodiments shown and described herein.
Fig. 6H shows a perspective view of a motion controller according to one or more embodiments shown and described herein.
Fig. 7A shows a cross-sectional side view of a device for engaging a speculum according to one or more embodiments shown and described herein.
Fig. 7B shows a cross-sectional side view of a device for engaging a speculum according to one or more embodiments shown and described herein.
Fig. 7C shows the device of Fig. 7B in a second configuration.
Fig. 8A shows a side view of a steering mechanism according to some embodiments shown and described herein.
Fig. 8B shows a side view of another steering mechanism according to some embodiments shown and described herein.
Fig. 8C shows a top view of the steering mechanisms shown in Figs. 8A or 8B.
Fig. 8D shows a side view of a steering mechanism with a cable in a retracted position according to one or more embodiments shown and described herein.
Figs. 8E shows a perspective view of a third actuator according to some embodiments shown and described herein.
Fig. 8F shows a perspective view of the third actuator shown in Fig. 8E with the outer shell depicted as transparent.
Fig. 8G shows a perspective view of an inner element of the steering mechanism according to some embodiments shown and described herein.
Fig. 8H shows a perspective view of a steering mechanism according to some embodiments shown and described herein, with the outer shell illustrated as partially transparent.

### DETAILED DESCRIPTION

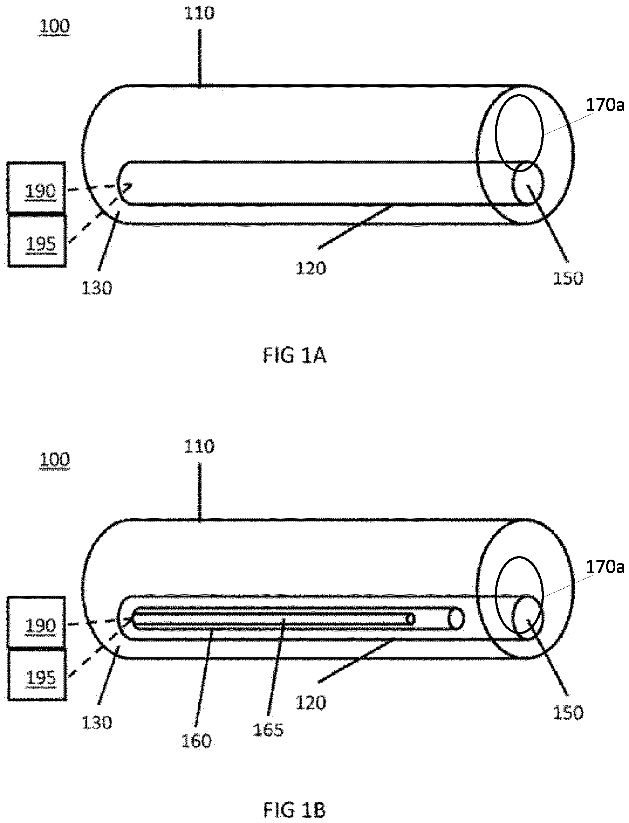

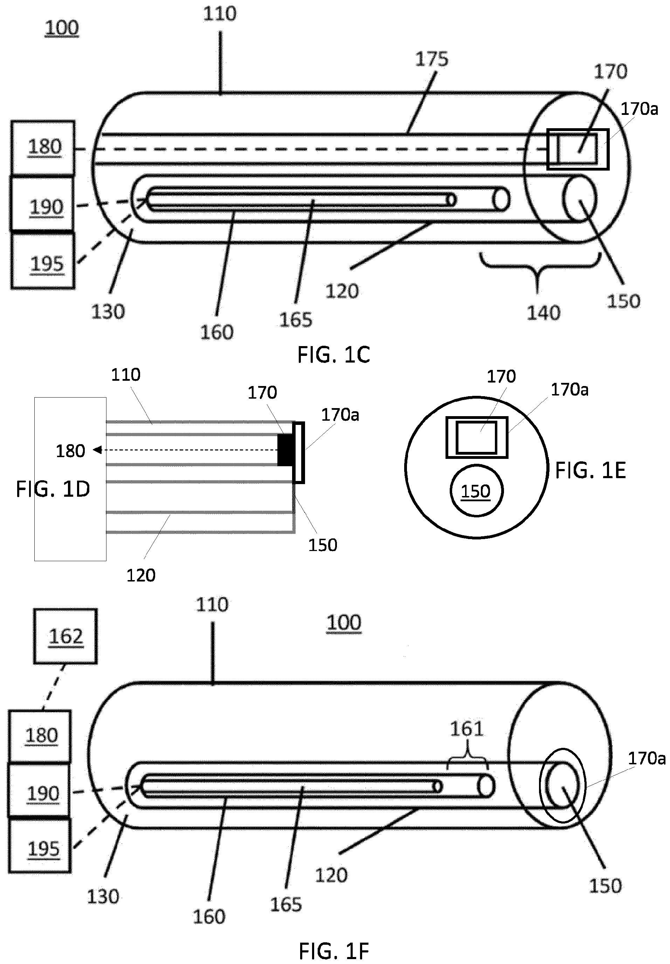

Fig. 1A shows a perspective view of an apparatus 100 suitable for delivering a fertilized egg or embryo into a maternal uterine endometrium, wherein the apparatus 100 comprises a body 110 (e.g. a catheter, also referred to as an outer body) configured to fit within a lumen of the female reproductive system. The body comprises one or more lumens including a lumen 120 extending from a proximal end 130 of the body 110 to a distal portion 140 of the body 110 and having a distal opening 150 at the distal portion 140 of the body. The lumen 120 is configured to slidably receive an inner body 160 having a distal end suitable for penetrating the endometrial epithelium. The apparatus 100 may also comprise a first actuator 190 operable to advance the inner body 160 out from the distal opening 150 of the body 110, and a second actuator 195 operable to expel a fertilized egg or embryo from the inner body.

The apparatus 100 may comprise a covering layer 170a at least partially covering the distal opening of one or more of the lumens (for example in the embodiment illustrated in Fig. 1A the covering layer 170a covers the distal opening 150 of lumen 120). The provision of a covering layer 170a inhibits mucus and other bodily fluids from entering the lumen 120 as the apparatus is advanced though the female reproductive system. This prevents components which are slidably received within one or more of the covered lumens (for example inner body 160 or a measurement assembly) from being exposed to the bodily fluids which may make them reusable when they are extractable from the apparatus 100. Furthermore, when the covering layer 170a is used for a lumen which contains a measurement assembly, it decreases the variability in measurements made by the measurement assembly to provide more reliable measurements for determining when the endometrial epithelium. For example, if the covering layer 170a is not provided, mucus and other bodily fluid may enter the lumen in an unpredictable manner and this may affect measurements made by the measurement assembly and therefore reduce the accuracy of determining when the endometrial epithelium has been reached. For example, when the measurement assembly comprises a capacitance sensor configured to make electrical contact with a distal portion of the inner body, it has been observed that the present of mucus in the lumen reduces the ability of the capacitance sensor to differentiate between the first state and the second state. Similarly, when the apparatus comprises a camera, mucus entering the lumen receiving the camera can cause the view of the camera to be partially or even completely obscured.

The dimensions of the body 110 are exaggerated in Fig. 1A - 1G for the purposes of clarity, whereas in reality the body 110 is a narrow, elongate body configured to extend into the uterus to the endometrial epithelium.

Fig. 1B shows an embodiment of the apparatus 100 of Fig. 1A, wherein the apparatus 100 further includes an inner body 160 having a distal end suitable for penetrating the endometrial epithelium. In this embodiment the inner body 160 is shown located in the lumen 120 of the body 110. A plunger 165 is housed within the inner body 160 and is configured to advance towards the distal end of the inner body 160 when actuated by the second actuator 195 and expel a fertilized egg which is located inside the inner body 160. The covering layer 170a may at least partially cover the distal opening 150 and the inner body 160 may be configured to perforate through the covering layer 170a when the inner body 160 is advanced out from the distal opening of the body, such that the inner body 160 can be advanced through the covering layer 170a and out of the distal opening 150 to penetrate the endometrial epithelium.

Fig. 1C shows a perspective view of another apparatus 100 suitable for delivering a fertilized egg or embryo into a maternal uterine endometrium, comprising a body 110 (e.g. a catheter, also referred to as an outer body) configured to fit within a lumen of the female reproductive system. Figs. 1D and 1E show side and front views of the distal portion of the apparatus 100 shown in Fig. 1C. The body 110 comprises one or more lumens including a lumen 120 extending from a proximal end 130 of the body 110 to a distal portion 140 of the body 110 and having a distal opening 150 at the distal portion 140 of the body. The lumen 120 is configured to slidably receive an inner body 160 having a distal end suitable for penetrating the endometrial epithelium. The apparatus 100 further comprises a measurement assembly comprising a measurement portion 170 disposed at the distal portion 140 of the body 110 (i.e. proximal to the distal opening 150). The measurement assembly 170 is configured to measure whether the apparatus is in a first state indicating the distance between the distal opening 150 of the body 110 and the endometrial epithelium is greater than a predetermined distance (i.e. in the direction that the inner body is advanced from the distal opening 150), or a second state indicating that the distance between the distal opening 150 of the body 110 and the endometrial epithelium is equal to or less than a predetermined distance. The apparatus 100 further comprises an indicating device 180 coupled to the measurement assembly 170 via a connection extending through a lumen 175 and configured to indicate that the measurement assembly 170 is in the first state or the second state. The measurement assembly may be removable from the body 110. In particular, the measurement portion 170 may be slidably received in a lumen of the body such that it may be extracted from the body 110 through the lumen, meaning the measurement assembly may be reusable. The inner body 160 and the measurement assembly may be received in the same or different lumens. The apparatus 100 also comprises a first actuator 190 operable to advance the inner body 160 out from the distal opening 150 of the body 110 by at least the predetermined distance, and a second actuator 195 operable to expel a fertilized egg from the inner body (for example to advance a plunger 165). The covering layer 170a at least partially covers at least one of the distal openings of the lumens. When the covering layer 170a covers the distal opening of the lumen 175 and thus the measurement portion 170, the covering layer prevents exposure of the measurement portion 170 to mucus and increases its reusability when the measurement assembly is removable from the apparatus (i.e. in embodiments where the measurement assembly is not fixedly attached to the inner lumen 175 and this removable).

In some embodiments, the measurement portion 170 is a camera proximal to the opening 150 and configured to view a portion of the endometrial epithelium.

The covering layer 170a preferably comprises a biocompatible transparent or semi-transparent film, and more preferably comprises or consists of polyolefin, PVC, paraffin, cellulose and derivates, hyaluronic acid thin films, parylene C-films, collagen, gelatin, vegetable casings, alginatesor composites or combinations thereof. Whilst the covering layer 170a would be expected to reduce the clarity of the image of the endometrial epithelium, it actually provides a clearer image than when mucus is allowed to enter into the lumen 175 and obscure the camera.

The predetermined distance may be, for example, less than 6mm. The inner body may be slidable to extend from the distal opening by any suitable distance, determined by the predetermined distance to the endometrial epithelium and the desired implantation depth of the embryo. For example, the inner body 160 may be slidable to extend from 0 to 6mm from the distal opening 150 of the body 110.

The body 110 may be an elongate body made of any suitable flexible and biocompatible material, such as PTFE, FEP, PEEK or other flexible lubricious material. Elongate body 110 and lumens may be formed, for example, by extrusion using known methods. The outer diameter of the body 110 may be between 1mm and 1.6mm, more preferably between 1.2mm and 1.4mm, and even more preferably 1.3mm. The total length of the body 110 may be any suitable length for insertion to a position proximal to the endometrial epithelium. For example, the body 110 may have a length of 400mm to 500mm, for example 450mm.

The inner body 160 may be made of any suitable biocompatible material such as PEEK and PI (Polyimide). Such materials enable the walls of the inner body 160 to be made relatively thin whilst maintaining the required rigidity of the inner body 160 for penetrating the endometrial epithelium. The inner body 160 may have any suitable outer diameter which slidably fits within the lumen 120 of the body 110. For example, the lumen 120 may have an inner diameter of greater than 0.45mm and the outer diameter of the inner body may be about 0.45mm.

The plunger 165 may also be made of materials such as PTFE, FEP, PEEK or other flexible lubricious material. The plunger 165 may have a suitable diameter which closely matches the inner diameter of the inner body 160 to effectively aspirate or expel fluid from the inner body 160. For example, the inner diameter of the inner body and the diameter of the plunger 165 may be about 0.28mm.

The measurement assembly may be any suitable measurement assembly that enables it to be determined whether the distance between the distal opening 150 of the body 110 and the endometrial epithelium is greater or less than the predetermined distance. It is noted that the measurement assembly may provide a qualitative measurement (for example the measurement assembly may transition between two states) or a quantitative measurement (for example actually measuring a parameter which indicates a value of the distance to the endometrial epithelium and comparing the parameter to a threshold value indicating the predetermined distance). The measuring portion 170 is the part of the measurement assembly which is configured to interact with the endometrial epithelium in order for the measurement assembly to make the determination and may comprise any suitable element or elements. The elements of the measuring portion 170 may be connected to components at the proximal end of the apparatus 100, such as components of the measurement assembly (not shown) and the indicating device 180 via connections extending through one or more lumens 175 in the body 110. For example, the measuring portion may comprise components optically, acoustically, or electrically connected to proximal components of the measurement assembly such as light or sound emitters or receivers, electrical signal components or electrical power sources. The measurement assembly may be any of the measurement assemblies disclosed in PCT application number PCT/EP2021/078712, which is incorporated herein in its entirety by reference.

The indicating device 180 is coupled to the measurement assembly insofar as the difference between the two states of the measurement assembly can be determined (i.e. functionally coupled). The indicating device 180 may be any suitable device which can indicate the difference between the two states. For example, if the measurement assembly provides a qualitative visual difference between the two states, then the indicating device 180 may be a display (e.g. scope display or digital display) configured to display the visual difference to a user of the apparatus, or it may comprise suitable software and/or hardware to receive data (such as image data) from the measurement assembly and perform analysis of the data to determine the state of the measurement assembly (or display the image data). The result of the analysis may be displayed. Alternatively, the measurement assembly may comprise the software/hardware and provide the result of the analysis to the indicating device 180.

The indication provided by the indicating device 180 may be provided to a user of the apparatus 100 if the first actuator 190 is to be actuated manually by the user (for example a visual indication on a display or an audio indication from a speaker), or it may be provided as a command signal to an electronic controller (such as the motion controllers disclosed herein) if the first actuator 190 is to be actuated automatically by the electronic controller. The indication provides a means for the operator or controller to verify that the distal opening is close enough to the endometrial epithelium to deliver the fertilized egg through the inner body.

The first and second actuators 190, 195 may be any suitable actuator controllable to advance the inner body 160 and to expel the fertilized egg. For example, the first actuator 190 may be a pusher or other manual tool for advancing the inner body, or it may comprise a motor system configured to advance the inner body. Likewise, the second actuator 195 may be configured to slide a plunger movable in the inner body to pressurize fluid in the inner body distally and expel the egg. The second actuator may comprise a lead screw for advancing the plunger, i.e. the second actuator may be actuated by applying a torque, and the resulting rotary motion converted into linear motion of the plunger. The rotary actuation of the lead screw allows for a high controllability of the linear motion, and thus the aspirated and dispensed liquid volumes.

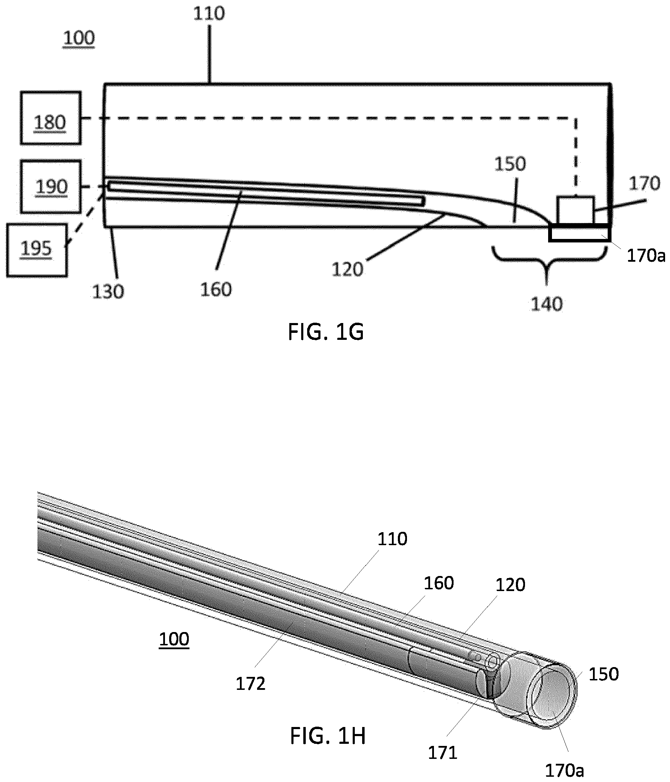

Whilst Fig. 1D shows the measurement portion 170 and distal opening 150 of the body 110 at the distal tip of the body 110, in other embodiments the measurement portion 170 and distal opening of one or more of the lumens such as distal opening 150 may be located at a side of the distal portion 140 of body 110, as shown in Fig. 1G. The lumen 120 comprises a gradually curved section proximal to the distal opening 150 of the body 110 in order to guide the inner body 160 laterally. The covering layer 170a may be provided at least partially on a sidewall of the body 110 to cover one or more distal openings of one or more lumens on a sidewall of the body 110.

Fig. 1C shows the measurement assembly comprising a measurement portion 170 disposed at the distal portion 140 of the body 110. In other embodiments the measurement portion 170 may be located at a different location (e.g. at a distal portion of the inner body 160) or the measurement assembly may be configured to interact with an element of the apparatus 100 in order to provide a qualitative or a quantitative measurement and may comprise any suitable element or elements, without the need of having a measurement portion within the measurement assembly. This is shown in the embodiment of Fig. IF, wherein the apparatus 100 comprises an inner body 160 having a distal portion 161 comprising a distal end suitable for penetrating the endometrial epithelium. The apparatus 100 further comprises a body 110 (e.g. a catheter) configured to fit within a lumen of the female reproductive system. The body comprises a lumen 120 extending from a proximal end 130 of the body 110 to a distal portion 140 of the body 110 and having a distal opening 150 at the distal portion 140 of the body. The lumen 120 is configured to slidably receive the inner body 160. The apparatus 100 of Fig. IF further comprises a measurement assembly 162. The measurement assembly 162 is configured to measure whether the apparatus 100 is in a first state indicating the distance between the distal end of the inner body 160 and the endometrial epithelium is greater than a predetermined distance (i.e. in the direction that the inner body is advanced from the distal opening 150 of the body 110), or a second state indicating that the distance between the distal end of the inner body 160 and the endometrial epithelium is equal to or less than a predetermined distance. The apparatus 100 of Fig. IF further comprises an indicating device 180 coupled to the measurement assembly 162 and configured to indicate that the apparatus 100 is in the first state or the second state. The apparatus 100 also comprises a first actuator 190 operable to advance the inner body 160 out from the distal opening 150 of the body 110, and a second actuator 195 operable to expel a fertilized egg from the inner body (for example to advance a plunger 165).

In an embodiment, the distal portion 161 of the inner body 160 is electrically conductive, and the measurement assembly 162 is configured to make electrical contact with such distal portion 161 of the inner body 160. The measurement assembly thus provides an indication of the capacitance. In an embodiment, the first state is indicated by a capacitance measurement below a threshold value, and the second state is indicated by a capacitance measurement above the threshold value. The covering layer 170a may cover the distal opening 150 to prevent mucus from entering the lumen 120 and affecting the capacitance measurements of the distal portion 161 as the inner body 160 is moved towards the distal opening 150. The inner body 160 is configured to perforate the covering layer 170a so that it can be extended distally from the apparatus 100 to penetrate the endometrial epithelium and transfer the fertilized egg.

In a particular embodiment, the capacitance varies according to the variation of the distance between the distal portion 161 of the inner body 160 and the endometrial epithelium, i.e. the capacitance value changes depending on the proximity of the distal portion 161 of the inner body 160 with the endometrial epithelium. In an embodiment, the predetermined distance is zero (thus meaning contact between the distal portion 161 of the inner body 160 and the endometrial epithelium). In an embodiment, the capacitance value is measured by means of the measurement assembly 162, which comprises an electrical system which is electrically connected to the distal portion 161 of the inner body 160 for measuring the capacitance.

The use of the measurement assembly in combination with the indicating device assists in determining that the distal portion of the apparatus is within a predetermined distance such that the inner body can be advanced into the endometrial epithelium to deliver the fertilized egg. In embodiments wherein the indication provides information regarding the distance between the distal portion of body 110 and the endometrial epithelium, this may assist in avoiding direct contact between the distal portion of body 110 and the endometrial epithelium. Further, prematurely extending the inner body 160 into the uterus whilst the apparatus is still being positioned could cause damage to the inner body 160. Accordingly, the indicating device assists in identifying when it is appropriate to extend the inner body 160 out from the apparatus.

In embodiments wherein the indication provides information regarding the distance between the distal end of the inner body 160 and the endometrial epithelium, the use of the measurement assembly in combination with the indicating device assists in determining that the distal end of the inner body is within a predetermined distance such that the inner body can be further advanced into the endometrial epithelium to deliver the fertilized egg and/or that the inner body is in a position wherein the fertilized egg can be delivered. Accordingly, the indicating device assists in identifying when it is appropriate to deliver the fertilized egg.

In an embodiment, the apparatus 100 of Figs. 1A - IF further comprises an imaging device, in particular a camera, not shown in the figures. In an embodiment, the camera is attached to the body 110 and moves together, as an integral device, with said body 110. In a particular embodiment, the camera is housed within the body 110. Fig. 1H shows a perspective view of a distal portion of an apparatus 100 comprising a camera 171 and an inner body 160 slidably received in a single lumen 120. The apparatus of Fig. 1H may comprise all of the features shown in Fig. IF with the addition of the camera 171 provided in the same lumen 120. The covering layer 170a covers the distal opening 150 of the lumen 120. The camera 171 may be comprised on an elongate body 172 comprising a longitudinal recess for receiving the inner body 160. The camera 171 may be connected to an display device (not shown) for displaying the field of view of the camera 171 via electrical connections extending through the elongated body 172.

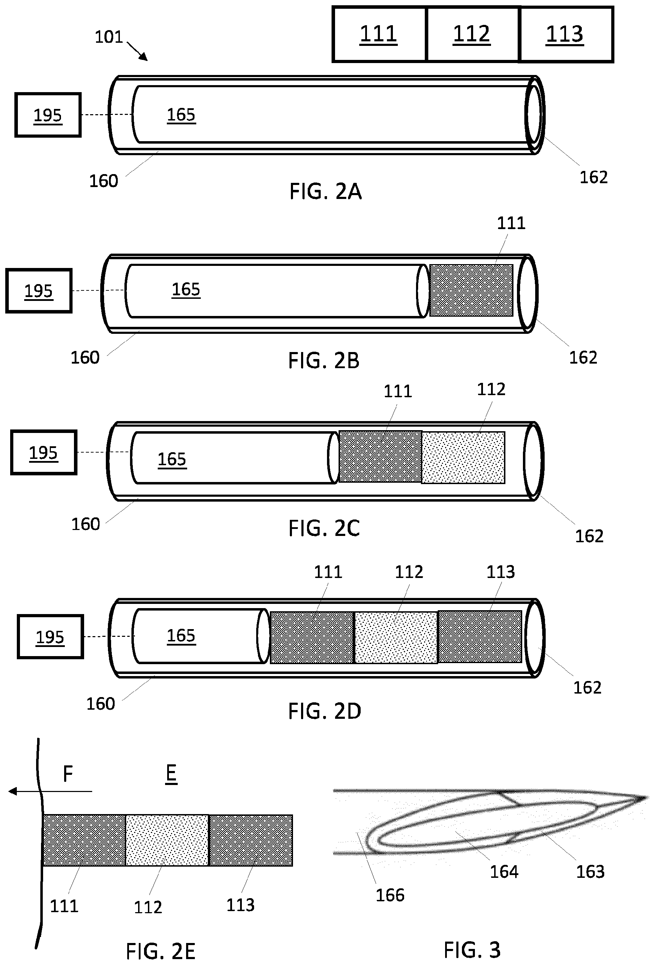

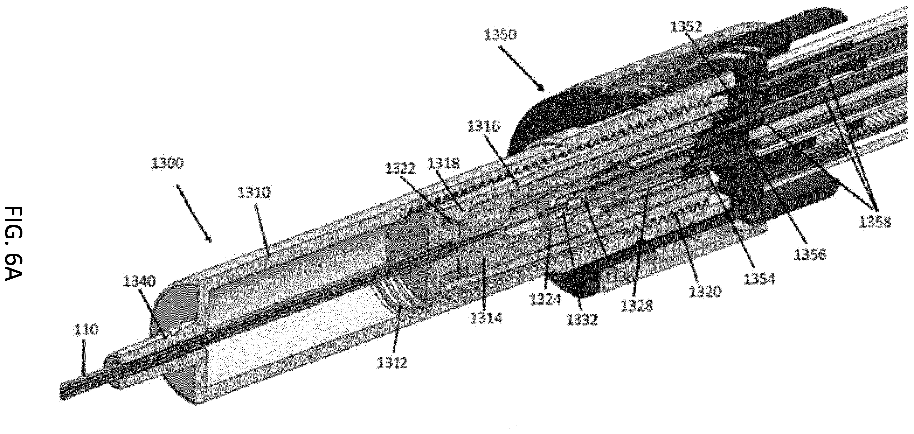

Fig. 2A shows a system 101 for delivering a fertilized egg or embryo into a maternal uterine endometrium in humans or any other mammalian species. The system 101 comprises a body 160 having a distal end 162 suitable for penetrating the endometrial epithelium. The system 101 further comprises a plunger 165 slidably received in a lumen of the body 160, and an actuator 195 operable advance the plunger 165 to expel a fertilized egg from the lumen of the body 160. The actuator 195 may be any suitable actuator for advancing and retracting the plunger 165, and may be a manual actuator such as a proximal end of the plunger 165 which remains accessible to a user when the system 101 is inserted into a lumen of the female reproductive system. The actuator 195 may be any of the actuators disclosed herein any may be a lead screw rotatable to advance and retract the plunger 165 as disclosed with reference to Figs. 6A to 6H. The system 101 further comprises a first injectable medium 111 and a second injectable medium 112 for containing a fertilized egg. The first injectable medium 111 has a higher viscosity than the second injectable medium 112.

The first injectable medium 111 may be any suitable bio-compatible injectable medium that has a higher viscosity than the second injectable medium 112. The second injectable medium 112 may be any injectable medium which is biocompatible and non-toxic to the fertilized egg, as known in the art. It will be understood that the lower bound for the viscosity of the first injectable medium 111 will depend on the viscosity of the second injectable medium 112 selected. In some embodiments, the first injectable medium 111 comprises or consists of one or more of the following: hyaluronic acid, a salt of hyaluronic acid, a biocompatible hydrogel, poloxamer, collagen, fibrinogen, gelatin, sodium hyaluronate and combinations thereof. Use of hyaluronic acid may promote the repair of the endometrial epithelium at the perforation sit of the endometrial epithelium by the body 160.

In some embodiments, there is also provided a third injectable medium 113 also having a higher viscosity than the second injectable medium 112. The third injectable medium 113 may be the same or different viscosity than the first injectable medium 111 and may comprise or consist of the same or different substances to the first injectable medium 111. In some embodiments, the third injectable medium 113 comprises or consists of one or more of the following: hyaluronic acid, a salt of hyaluronic acid, a biocompatible hydrogel, poloxamer, collagen, gelatin, fibrionogen, sodium hyaluronate and combinations thereof.

The first injectable medium 111 and second injectable medium 112 (and optionally the third injectable medium 113) may be provided in medium reservoirs (for example vials or similar) and a user may manually load the system 101 with the injectable mediums by inserting the body 160 into the reservoirs and retracting the plunger 165 to draw in each injectable medium into the lumen of the body 160. In some embodiments, the injectable mediums may be preloaded in the body 160.

In one embodiment, the first injectable medium 111 and the second injectable medium 112 are received (i.e. pre-loaded) in the lumen of the body 160, wherein the second injectable medium 112 is located ahead of the first injectable medium 111 relative to the distal end 162 of the body 160 such that the second injectable medium 112 is expelled before the first injectable medium 111 when the actuator 195 is actuated to expel a fertilized egg. Fig. 2C shows such a configuration. As discussed later in further detail with reference to Fig. 2E, the first injectable medium 111 acts as a plug inhibiting the second injectable medium from being expelled from the endometrial epithelium E.

In one embodiment, the first injectable medium 111, the second injectable medium 112 and the third injectable medium 113 are received (i.e. pre-loaded) in the lumen of the body 160, wherein the second injectable medium 112 is received between the first and third injectable mediums 111, 113, such that the second medium 112 is expelled before of one of the first and third injectable mediums 111, 113, and after the other of the first and third injectable mediums, 111, 113, when the actuator 195 is actuated to expel a fertilized egg. Fig. 2D shows such a configuration. As discussed later in further detail with reference to Fig. 2E, the first injectable medium 111 acts as a plug inhibiting the second injectable medium from being expelled from the endometrial epithelium E and third injectable medium 113 provides additional structural support to maintain the second injectable medium 112 containing the fertilized egg in the desired injected position within the endometrial epithelium E.

The system 101 illustrated in Fig. 2A (and in any of the configurations shown in any of Figs. 2A to 2D) may be provided as shown in Fig. 2A or may be incorporated into any suitable embryo transfer system. For example, the body 160 may be slidably received through a delivery catheter which is positionable within a lumen of the female reproductive system. The system 101 may optionally be incorporated in any of the embryo transfer systems disclosed herein. In particular, the body 160 shown in Figs. 2A to 2D may be the inner body 160 shown in any of Figs. 1B to 1H, and may additionally comprise an body 110 (also referred to as an outer body) configured to fit within a lumen of the female reproductive system, the body 110 comprising an outer lumen 120 extending from a proximal end 130 of the body 110 to a distal portion 140 of the body 110 and having a distal opening 150 at the distal portion 140 of the body 110, the lumen 120 configured to slidably receive the body 160 (also referred to as an inner body). The system may additionally comprise an actuator 190 operable to advance the inner body 160 out from the distal opening 150 of the outer body 110.

A method of loading the system 101 of Fig 2A (whether incorporated into the system described with respect to Figs. 1B to 1H or any other embryo transfer system) may be as follows. In a first step, the first injectable medium 111 may be drawn into the lumen of the body 160. In particular, the body 160 may be inserted into a reservoir containing the first injectable medium 111 and the actuator 195 may be actuated to retract the plunger 165 in order to draw the first injectable medium 111 into the lumen of the body 160 (Fig. 2A to Fig. 2B). In a second step subsequent the first step, the second injectable medium 112 may be drawn into the lumen of the body 160. In particular, the body 160 may be inserted into a reservoir containing the second injectable medium 112 and the actuator 195 may be actuated to retract the plunger 165 in order to draw the second injectable medium 112 into the lumen of the body 160 (Fig. 2B to Fig. 2C). In some embodiments, in a third step subsequent the second step, the third injectable medium 113 may be drawn into the lumen of the body 160. In particular, the body 160 may be inserted into a reservoir containing the third injectable medium 113 and the actuator 195 may be actuated to retract the plunger 165 in order to draw the third injectable medium 113 into the lumen of the body 160 (Fig. 2C to Fig. 2D).

It is noted that the system 101 may be delivered to the endometrial epithelium E in either of the loaded states shown in Figs. 2C and 2D and the actuator 195 may be actuated to deliver the first and second injectable mediums 111 and 112 to the endometrial epithelium E. Fig. 2E shows the injectable mediums 111, 112 and optionally 113 inside the endometrial epithelium E after injection by the system 101. When the system is removed from the endometrial epithelium E after injection, it has been observed that the endometrial epithelium E exerts an expulsive force F on the injected substance and the injectable medium 112 is at risk of being expelled from the endometrial epithelium E immediately after injection, risking a failed fertility treatment procedure. Provision of the first injectable medium 111 in the position shown in Fig. 2B allows the first injectable medium 111 to be the outermost part of the injected substance in the endometrial epithelium E, as shown in Fig. 2E, and provides a high viscosity plug which better resists the expulsive force F and therefore reduces the risk of the endometrial epithelium E expelling the second injectable medium 112 from the endometrial epithelium E. In embodiments where the third injectable medium 113 is used, provision of the third injectable medium 113 in the position shown in Fig. 2D allows the third injectable medium 112 to be the innermost part of the injected substance in the endometrial epithelium E, as shown in Fig. 2E. The first and third injectable mediums 111, 113, therefore provide a pocket within the endometrial epithelium E where the second injectable medium 112 is located, with the first and third injectable mediums 111, 113 providing structural support either side of the pocket to inhibit transmission of the forces, such as the expulsive force F or any inward pressure from the surrounding tissue, to be transmitted to the second injectable medium 112, thereby further increasing the stability of the injected substance when situated in the endometrial epithelium E.

In any of the embodiments disclosed herein with reference to Figs. 1A to 2E, the inner body 160 may comprise a hydrophobic coating formed on a distal portion of the inner body 160. The hydrophobic coating may be formed on one, two or all of an inner surface 164 of the distal portion of inner body 160, the distal tip 163 of the inner body 160 and the outer surface 166 of the distal portion of the inner body 160. Alternatively, or additionally, the inner body 160 may comprise a bevelled tip 163.

When a covering layer is provided covering the distal opening of the lumen containing the inner body 160, the bevelled tip 163 assists in perforating the covering layer. Further, the bevelled tip 163 decreases the force required to penetrate the endometrial epithelium.

The hydrophobic coating may comprise or consist of a polymer or polymer composite, preferably any of the following: parylene, acrylic, polyethylene, polyurethane and polytetrafluorethylene and composites thereof. The hydrophobic coating inhibits sticking of the fertilized egg or embryo to the distal portion of the inner body 160 (for example resulting in the embryo or egg not being expelled in the first place, or being extracted from the endometrial epithelium with the inner body 160) and therefore reduces the risk of a failed egg or embryo transfer procedure.

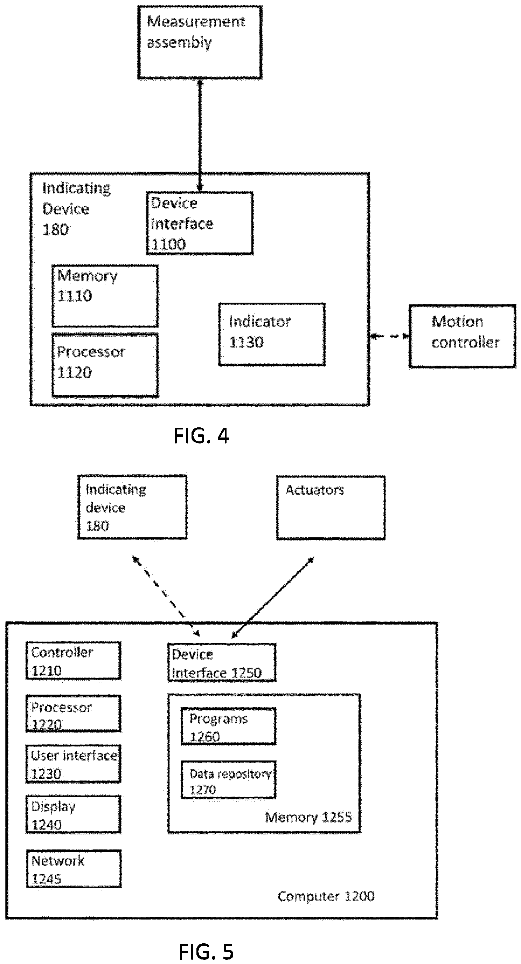

Fig. 4 shows a diagram of an indicating device 180 which may be used for any of the apparatuses disclosed herein comprising a measurement assembly, when the output of the measurement assembly is a signal (for example if the measurement assembly comprises a camera or distance measuring probe such as a capacitance sensor). The indicating device comprises a device interface 1100 configured to communicate with (i.e. send and receive signals from) the measurement assembly. The indicating device further comprises a memory 1110, processor 1120 and indicator 1130. It will be appreciated than any suitable computing system may be used for the indicating device 180 and that the device shown in Fig. 4 is one of many devices that could be used. For example, a distributed computer system may be used, for example comprising one or more servers or client computing systems, using any known distributed computing technique. In some examples, a general-purpose computer or any other processing system may be used to implement the methods disclosed herein. Furthermore, the steps disclosed below may be implemented in software, hardware, or any combination of these to achieve the same steps.

It will be appreciated by the skilled person that the indicating device of Fig. 4 may be powered by any suitable powering means. The memory 1110 may comprise one or more volatile or non-volatile memory devices, such as DRAM, SRAM, flash memory, read-only memory, ferroelectric RAM, hard disk drives, floppy disks, magnetic tape, optical discs, or similar. Likewise, the processor 1120 may comprise one or more processing units, such as a microprocessor, GPU, CPU, multi-core processor or similar. The device interface 1100 may comprise wired connections, such as optic, fiber-optic, ethernet or similar, or any suitable wireless communication.

In the case of the measurement assembly making a quantitative measurement (e.g. comprising a distance sensing probe), the memory 1110 may contain suitable instructions for performing a comparison of the received signal to the predetermined distance, and the processor 1120 may be configured to perform the instructions. For example, the indicating device 180 may receive the raw data signal via device interface 1110, the processor 1120 may convert the raw data into a numerical value for the predetermined distance, and the numerical value may be compared to the predetermined distance. If the numerical value is equal to or lower than the predetermined distance, then the processor 1120 may instruct the indicator 1130 to indicate that the predetermined distance has been reached. For example, the indicator 1130 may provide an audio or visual signal to the operator, or may comprise an electronic controller configured to provide a command signal to an electronically controlled motion controller to perform the operation of advancing the needle and expelling the embryo (with the memory 1110 containing suitable instructions for providing control signals to the actuators). The indicating device 180 may also be configured to further indicate to the user when the automatic procedure of advancing the need and expelling the embryo has been completed (for example an audio or visual signal). Alternatively, the indicator 1130 may comprise a display displaying the measured distance alongside the predetermined distance.

In the case of the measurement assembly comprising a camera and the measurement being a qualitative measurement, the indicating device may merely be a display for displaying the real-time image captured by the camera, optionally with a comparison overlay on the display where appropriate. Alternatively, the memory 1110 may store instructions for performing image analysis of the image data to automatically determine if the image indicates that the predetermined distance has been reached. The processor 1120 may be configured to carry out the instructions. The processor 1120 may then instruct the indicator 1130 to indicate that the predetermined distance has been reached. For example, the indicator 1130 may provide an audio or visual signal to the operator, or may provide a command signal to an electronically controlled motion controller to perform the operation of advancing the needle and expelling the embryo.

Whilst Fig. 4 shows the motion controller and the indicating device as separate devices, indicating device and motion controller may be incorporated into a single device. Further, the measurement assembly may instead be connected to the indicating device indirectly via a device interface of the motion controller.

Fig. 5 shows a diagram of a computer 1200 that may be used to control one or more of the actuators disclosed herein. The computer 1200 may comprise one or more of a controller 1210, a processor 1220, a user interface 1230, a display 1240, a network connection 1245, a device interface 1250, and a memory 1255 storing instructions for programs 1260 and a data repository 1270. It will be appreciated than any suitable computing system may be used for the computer 1200 and that the device shown in Fig. 5 is one of many devices that could be used. For example, a distributed computer system may be used, for example comprising one or more servers or client computing systems, using any known distributed computing technique. In some examples, a general-purpose computer or any other processing system may be used to implement the methods disclosed herein. Furthermore, the steps disclosed below may be implemented in software, hardware, or any combination of these to achieve the same steps.

It will be appreciated by the skilled person that the indicating device of Fig. 5 may be powered by any suitable powering means. The memory 1255 may comprise one or more volatile or non-volatile memory devices, such as DRAM, SRAM, flash memory, read-only memory, ferroelectric RAM, hard disk drives, floppy disks, magnetic tape, optical discs, or similar. Likewise, the processor 1220 may comprise one or more processing units, such as a microprocessor, GPU, CPU, multi-core processor or similar. The device interface 1250 may comprise wired connections, such as optic, fiber-optic, ethernet or similar, or any suitable wireless communication.

The device interface 1250 is configured to communicate with (i.e. send and receive control information) one or more electronically controlled actuators. Any of the actuators disclosed herein may be electronically controlled. The device interface 1250 may also be configured to communicate with the indicating device 180. Alternatively, the computer 1200 may comprise all of the features of the indicating device shown in Fig. 4 and may itself be considered as the indicating device 180.

The computer 1200 provides manual control of the electronically controlled actuators by a user of the apparatus. For example, the memory 1255 may comprise instructions for a program 1260 which when executed by processor 1220 causes a graphical user interface (GUI) to be displayed on display 1240, or remotely via network connection 1245. The GUI may comprise appropriate inputs to enable the user to actuate one or more of the actuators. For example, the user may select a function from the GUI, which causes the controller 1210 to send a corresponding control signal to one or more actuators to perform the function.

For example, the user may be able to select a command from the GUI for advancing the body by a certain distance, or at a certain constant speed. The user may be able to select one or more commands for steering the steering mechanism. The user may be able to select one or more commands for advancing the inner body and/or plunger. The commands may correspond to a sequence of commands such that several commands are performed in sequence. For example, the memory 1255 may store instructions for advancing the body 110 until the predetermined distance is reached (i.e. until the indicating device indicates that the predetermined distance is reached), and for subsequently advancing the inner body and plunger to expel the embryo. It may be that all actuators are actuated via such an automated process, or that only some are actuated automatically and the rest are actuated manually (i.e. by user control via the GUI).

The computer 1200 may be configured to receive data from the measurement assembly, either directly or via indicating device 180, and display the data on the GUI alongside the command options. For example, the display 1240 may display real-time image data received from the measurement assembly. The GUI may also display an indication that the predetermined distance has been reached to the user.

In some embodiments, the apparatus may further comprise a motion controller configured to connect to a proximal portion of the body 100, wherein the controller is configured to actuate the first actuator, second actuator, and/or a fourth actuator for advancing the body relative to the motion controller. Any suitable motion controller may be used. For example, the motion controller may comprise one or more linear or rotary mechanisms for actuating the first actuator, the second actuator and/or the fourth actuator. In some embodiments, one or more of the first, second and fourth actuator may comprise a lead screw, each lead screw rotatable to advance the body 110, inner body 160 or plunger 165, the motion controller may be configured to actuate the one or more lead screws. The motion controller may be manually controlled or electronically controlled. The motion controller may be configured to connect to the body via a connector.

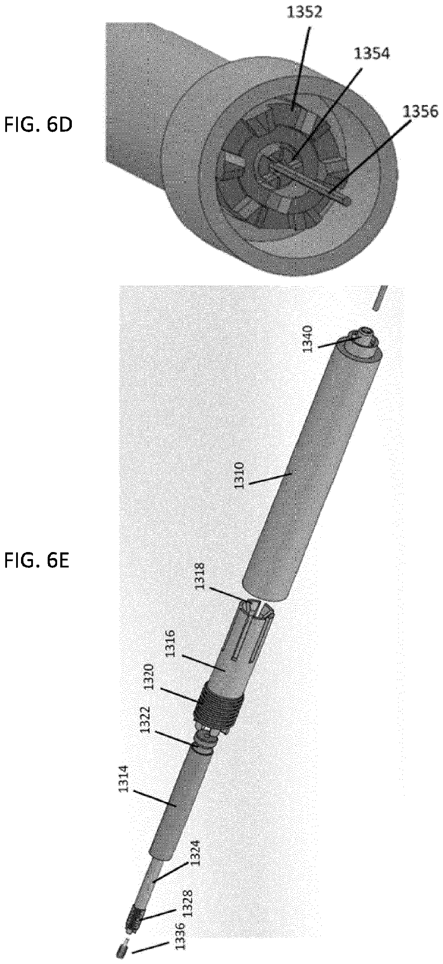

Fig. 6A shows a cross-sectional perspective view of a connector 1300 configured to connect to a motion controller 1350. Fig. 6E shows an exploded view of the connector 1300. The connector 1300 comprises a connector housing 1310 which is insertable into a distal end of the motion controller 1350. The connector 1300 also comprises a body connector 1314 fixedly receiving a proximal portion of the body 110 (e.g. by a frictional fit or by adhesive), an inner body connector 1324 fixedly receiving a proximal portion of the inner body 160 (e.g. by frictional fit or by adhesive) and a plunger connector 1332 fixedly receiving a proximal portion of the plunger 165 (e.g. by frictional fit or by adhesive).

The body connector 1314 is received by the connector housing 1310 and is slidable relative to the connector housing 1310 at a fixed rotational orientation. For example, the connector housing 1310 may comprise a longitudinally extending protrusion or recess and the body connector 1314 may comprise a corresponding recess or protrusion such that the body connector 1314 is only receivable in the connector housing 1310 at a single rotational orientation. The connector 1300 further comprises a body lead screw 1316 having an external screw thread 1320 which corresponds to an internal screw thread 1312 of the connector housing 1310. The body lead screw 1316 abuts the body connector 1314 such that, when turning the body lead screw 1316 to advance the body lead screw distally, the body connector 1314 also advances distally (but in a fixed orientation). In the illustrated embodiment, the body lead screw 1316 is connected to the body connector 1314 by one or more protrusions 1318 which are received by a corresponding recess 1322 in the body connector. The interaction between the protrusion 1318 and the recess 1322 means that the body lead screw 1316 abuts the body connector 1314 when it slides both distally and proximally, meaning the body lead screw 1316 is able to both advance and retract the body connector 1314 and thus the body 110. In some embodiments the protrusion 1318 may instead be located on the body connector 1314 and the recess on the body lead screw 1316.

The inner body connector 1324 is housed by the body connector 1314 and is slidable relative to the connector housing 1310 and the body connector 1314. In some embodiments, the inner body connector 1324 may be slidable relative to the connector housing 1310 and body connector 1314 at a fixed rotational orientation (for example using corresponding protrusions and recessions in the body connector 1314 and the inner body connector 1324 as previously disclosed).

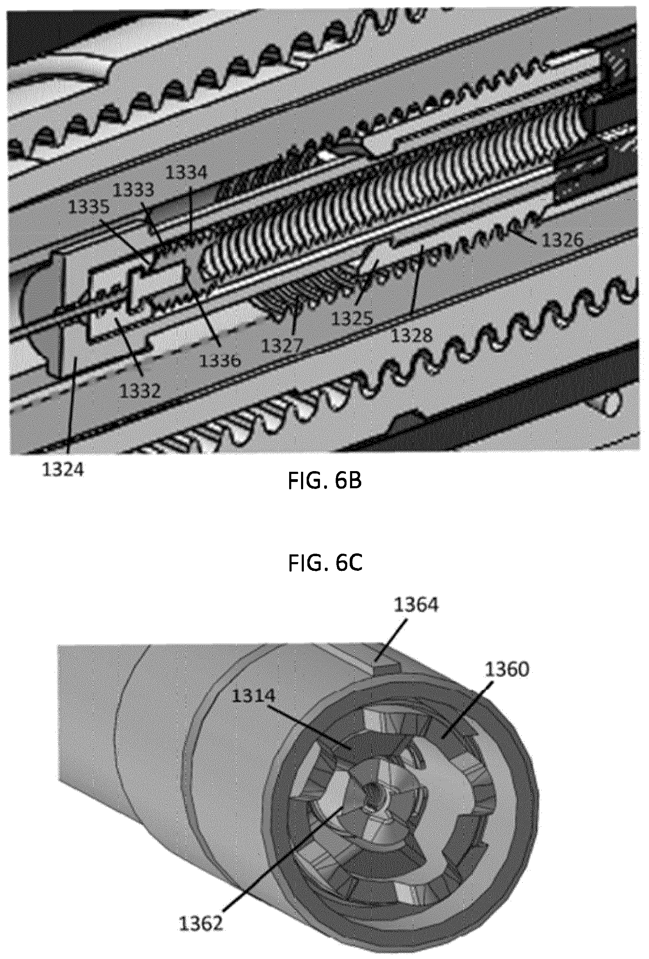

Fig. 6B shows a close up of the inner body connector 1324 and the plunger connector 1332 shown in Fig. 6A. The connector further comprises an inner body lead screw 1328 having an external screw thread 1326 which corresponds to an internal screw thread 1327 of the body connector 1314. The inner body lead screw 1328 abuts the inner body connector 1324 such that, when turning the inner body lead screw 1328 to advance the inner body lead screw 1328 distally, the inner body connector 1324 also advances distally. In the illustrated embodiment, the inner body lead screw 1328 is connected to the inner body connector 1324 by one or more protrusions 1325 which are received by a corresponding recess in the inner body connector 1325. The interaction between the protrusion 1325 and the recess means that the inner body lead screw 1328 abuts the inner body connector 1324 when it slides both distally and proximally, meaning the inner body lead screw 1328 is able to both advance and retract the inner body connector 1324 and thus the inner body 160. In some embodiments the protrusion 1325 may instead be located on the inner body connector 1324 and the recess on the inner body lead screw 1328. In some embodiments, the inner body connector 1324 and inner body lead screw 1328 may be a single unitary body.

The plunger connector 1332 is housed by the inner body connector 1324 and is slidable relative to the connector housing 1310, the body connector 1314 and the inner body connector 1324. In some embodiments, the plunger connector 1332 may be slidable relative to the connector housing 1310, body connector 1314 and inner body connector 1324 at a fixed rotational orientation (for example using corresponding protrusions and recessions in the inner body connector 1324 and the plunger connector 1332 as previously disclosed). The connector further comprises a plunger lead screw 1336 having an external screw thread 1333 which corresponds to an internal screw thread 1334 of the inner body connector 1324. The plunger lead screw 1336 abuts the plunger connector 1332 such that, when turning the plunger lead screw 1336 to advance the plunger lead screw 1336 distally, the plunger connector 1332 also advances distally. In the illustrated embodiment, the plunger lead screw 1336 is connected to the plunger connector 1332 by one or more protrusions 1335 in the plunger connector 1332 which are received by a corresponding recess in the plunger lead screw 1336. The interaction between the protrusion 1335 and the recess means that the plunger lead screw 1336 abuts the plunger connector 1332 when it slides both distally and proximally, meaning the plunger lead screw 1336 is able to both advance and retract the plunger connector 1332 and thus the plunger 165. In some embodiments the protrusion 1335 may instead be located on the plunger lead screw 1336 and the recess on the plunger connector 1332. In some embodiments, the plunger connector 1332 and plunger lead screw 1336 may be a single unitary body.

The connector 1300 may comprises an interlocking feature 1340 at a distal end, such as a Luer lock, for securing the distal end of the connector 1300 to the next distal component of the apparatus.

Fig. 6C shows a perspective view of a proximal end of the connector 1300. Fig. 6D shows a perspective view of a distal end of the motion controller 1350. The proximal end of the connector 1300 is configured to connect with the distal end of the motion controller 1350. The body lead screw 1316 comprises a toothed proximal end 1360 configured to engage with a body lead screw driver 1352 of the motion controller 1350. The inner body lead screw 1328 comprises a toothed proximal end 1362 which is configured to engage with an inner body lead screw driver 1354 of the motion controller 1350. The plunger lead screw comprises a toothed proximal end (not shown) configured to engage with a plunger lead screw driver 1356 of the motion controller 1350. The proximal end of the connector housing 1310 may further comprise a locking feature such as a protrusion 1364 configured to be received by a recess of the distal end of the motion controller (not shown) in order to prevent relative rotation of the distal end of the motion controller 1350 and connector housing 1310. The connector 1300 may be connected to the motion controller 1350 by a push-fit, click-fit, frictional-fit, or similar mechanism. The lead screw drivers 1352, 1354 and 1356 may be distally biased by springs 1358 (see Fig. 6A) in order to ensure engagement with the proximal ends of the lead screws during rotation.

Whilst the illustrated embodiment illustrates a connector which actuates the body connector, inner body connector and plunger connector by the lead screw mechanisms, it will be appreciated that in other embodiments the connector only actuates one or more of the body connector, inner body connector and plunger connector by a lead screw mechanism. For example, the connector may comprise only the body connector 1314, actuated by the body lead screw 1316 as described above, and the needle and plunger may be actuated differently (i.e. by a linear motion mechanism). It will also be appreciated that the outermost screw thread of a given embodiment engages the inner screw thread 1312 of the connector housing 1310. For example, in embodiments where the connector 1300 actuates only the inner body 160 and plunger 165 by the screw thread mechanisms disclosed, the outer thread 1326 of the inner body lead screw 1328 engages with the inner thread 1312 of the connector housing 1310, rather than the inner screw thread of the body connector 1314.

The connector housing 1310, connectors 1314, 1324 and 1332 and lead screws 1316, 1328 and 1336 may be made of any suitable material such as plastic and may be molded or 3D printed. Likewise, the lead screw drivers 1352, 1354 and 1356 may be made of any suitable material such as plastic.

Electrical wiring and/or optical fibers extending from the measurement portion 170 through the body 110 from the distal end of the apparatus may terminate at the connector 1300. In particular, a proximal portion of electrical wiring extending from the distal end of the apparatus through the body 110 may be electrically connected to an electrical terminal of the connector. The motion controller may comprise a corresponding electrical terminal biased towards the electrical terminal of the connector to maintain contact with the electrical terminal of the connector. Similarly, a proximal portion of the optical fibers may be optically connected to an optical terminal of the connector 1300, and the motion controller may comprise an optical terminal configured to form a face seal with the optical terminal of the connector 1300 and biased towards the optical terminal of the connector 1300 to maintain the face seal with the first optical terminal.

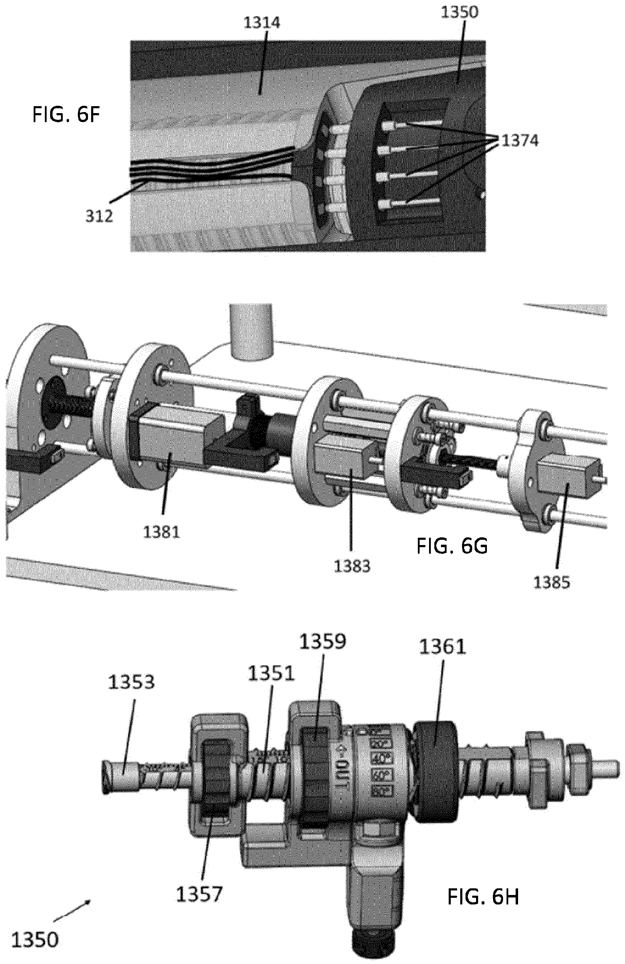

Fig. 6F shows a perspective view of the connection between the body connector 1314 and the distal end of the motion controller 1350. The illustrated embodiment shows a proximal portion of electrical cables 312 which extend from the measurement portion (e.g. camera 171), through the body 110 to the connector 1300. The cables 312 are electrically connected to an electrical terminal (such as a printed circuit board) on the body connector 1314 (e.g. by soldering). The electrical terminal comprises electrical connections which are configured to engage electrical pins 1374 on the motion controller 1350. The electrical pins 1374 are biased distally such that the electrical connection between the pins 1374 and the electrical terminal of the body connector 1314 is maintained as the body connector 1314 is advanced through the connector housing 1300. It will be appreciated that optical connections between the connector 1300 and the motion controller 1350 can be similarly established and maintained.

Fig. 6G shows a perspective view of a proximal portion of the motion controller 1350. The motion controller 1350 comprises a first motor 1381, a second motor 1383 and a third motor 1385. In one embodiment, the first motor 1381 may be configured to drive the body lead screw driver 1352, the second motor 1383 may be configured to drive the inner body lead screw driver 1354 and the third motor may be configured to drive the plunger lead screw driver 1356. The motion controller 1350 may be configured to advance/retract the body 110, inner body 160 and plunger 165 at the same rate by running the first, second and third motors 1381, 1383, 1385 in unison at the rate corresponding to the same advancement speed of the body 110, inner body 160 and plunger 165. The motion controller 1350 may also be configured to advance/retract the inner body 160 and plunger 165 at the same rate relative to the body 110 by running the second and third motors 1383, 1385 in unison at the rate corresponding to the same advancement speed of the inner body 160 and plunger 165. The motion controller 1350 may also be configured to advance/retract the plunger 165 relative to the inner body 160 by running the third motor 1385 alone.

Alternatively, the first motor 1381 may be configured to turn each of the lead screw drivers 1352, 1354 and 1356 (e.g. via a first threaded rod) to advance the lead screws 1316, 1328 and 1336 at the same rate (to advance the body 110, the inner body 160 and the plunger 165 at the same rate). The second motor 1383 may be configured to turn the inner body lead screw driver 1354 and the plunger lead screw driver 1356 (e.g. via a second threaded rod) to advance the inner body lead screw 1328 and the plunger lead screw 1336 at the same rate (to advance the inner body 160 and plunger 165 at the same rate). The third motor 1386 may be configured to turn the plunger lead screw driver 1356 (e.g. via a threaded rod) to advance the plunger 165.

The motion controller 1350 may further comprise an axial motor (not shown) to retract the plunger lead screw driver 1352 before connection or disconnection of the connector 1300, in order to prevent damage to the plunger lead screw driver 1352 during connection or disconnection of the connector 1300.

The connector 1300 may be assembled in the following steps (the steps need not necessarily be performed in chronologically in the order presented). The proximal portion of the plunger 165 may be fixed to the plunger connector 1332. The plunger connector 1332 may be connected to the plunger lead screw 1336 (for example the connector and lead screw are pushed together until the protrusion falls into the recess to secure the connector and lead screw together). The proximal portion of the inner body 160 may be connected to the inner body connector 1324. The inner body connector 1324 may be connected to the inner body lead screw 1328 (e.g. by pushing the connector and lead screw are together until the protrusion falls into the recess to secure the connector and lead screw together). The proximal portion of the body 110 may be connected to the body connector 1314. The body connector 1314 may be connected to the body lead screw 1316 (e,g. by pushing the connector and lead screw are together until the protrusion falls into the recess to secure the connector and lead screw together). The distal end of the plunger 165 may be passed through the inner body connector 1324 from the proximal end and through the inner body 160. The plunger lead screw 1336 may be screwed to the inner body connector 1324. The distal end of the inner body 160 may be passed through the body connector 1314 from the proximal end and through the body 110. The inner body lead screw 1328 may be screwed to the body connector 1314. The distal end of the body may be passed through the connector housing 1310 from the proximal end. The body lead screw 1316 may be screwed to the connector housing 1310. The lead screws may then be actuated to moved the body 110, inner body 160 and plunger 165 to the correct relative positions.

When the connector 1300 is assembled, the plunger lead screw 1336 may be actuated to aspirate the fluid containing the embryo. The connector 1300 is then connected to the motion controller 1350.

The length from the proximal end of the body connector 1314 to the distal tip of the body 110 may be about 300mm to 400mm, preferably 325mm to 375mm, for example 358mm. The maximum length of the connector 1300 from the proximal end to the distal tip of the body 110 may be bout 400mm to 500mm, preferably 425mm to 475mm, for example 450mm.

Fig. 6H shows a perspective view of a motion controller 1350 according to an embodiment. The motion controller 1350 is configured to connect to a proximal portion of the body 110 and to actuate on said body 110. In the embodiment shown in Fig. 6H, the motion controller 1350 comprises two lead screws 1351, 1353 configured to advance/retract the body 110, and to rotate and advance/retract an outer catheter which houses the body 110 in an embodiment. In the embodiment shown the motion controller 1350 further comprises three actuation mechanisms 1357, 1359, 1361, each actuation mechanism configured to actuate on one of the lead screws 1351, 1353. The actuation of the lead screws 1351, 1353 may be manual or may be motorized. Although in this embodiment two lead screws 1351, 1353 are shown, in other embodiments there may be only one lead screw, depending on the elements intended to be moved by the motion controller 1350. The motion controller 1350 allows fine and precise control of the movement of the body 110 and of the outer catheter housing the body, as well as fixing the outer catheter in the required position. In an embodiment, the motion controller 1350 may be actuated based on a real time image obtained by an imaging device, such as a camera, present in the apparatus 100.

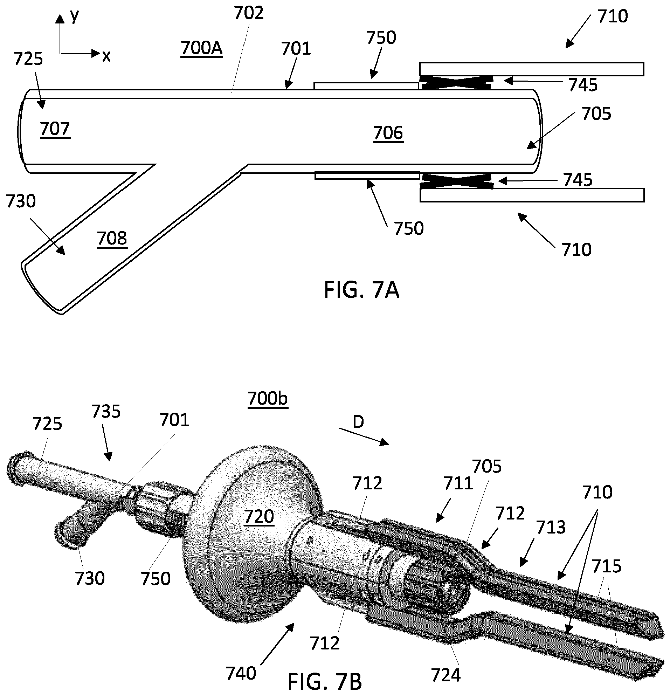

Fig. 7A shows a cross-sectional side view of a device 700a for engaging a speculum (also referred to as a speculum lock). The device 700a comprises two or more expandable engaging elements 710 for outwardly engaging a speculum to fix the device to a speculum, wherein the two or more expandable engaging elements 710 comprise a plurality of expanded configurations for engaging a plurality of speculum sizes. The device 700a comprises a guide 705 for guiding an embryo delivery device between the engaging element 710 and through a speculum when the device 700a is fixed to the speculum. In particular, the guide 705 at least partially conforms to an outer surface of the embryo delivery device to allow longitudinal movement of the embryo delivery device through the guide (i.e. in the x-direction) and to prevent lateral movement of the embryo delivery device in the guide (i.e. in the y-direction). The guide 705 may comprise a tubular body 701 having a tubular wall 702 defining a lumen 705 for slidably receiving the embryo delivery device, wherein the cross-sectional shape of the inner surface of the tubular wall 702 at least partially conforms to the outer surface of the embryo delivery device to allow longitudinal movement of the embryo delivery device through the guide and to prevent lateral movement of the embryo delivery device in the guide. The embryo delivery device may be the body 110 incorporated in any of the apparatuses for delivering a fertilized egg described with reference to Figs. 1A to 6H.

The expandable engaging elements 710 may be coupled to the tubular body 701. The engaging elements 710 may be expandable using any suitable mechanism. For example, the device 700a may comprise one or more biasing elements such as a spring connected between the tubular body 701 and the engaging elements 710 to bias the engaging elements 710 to an expanded configurations. The engaging elements 710 may be maintained in a collapsed configuration by a sheath or cover over the engaging elements 710. The engaging elements 710 may be inserted into the speculum and the sheath or cover may be removed (e.g. retracted) from the engaging elements 710 so that the engaging elements 710 are biased to the expanded configuration to engage opposing sides of the speculum to fix the device 700a to the speculum. Other mechanisms may be used, such as a motor connected to the engaging elements 710 for moving the engaging elements between a collapsed configuration and one or more expanded configurations. In some embodiments, the engaging elements 710 may be mounted to the guide 705 via respective scissor lifts 745. The scissor lifts 745 may comprise one or more pairs of struts pivotally connected to one another, with one strut being pivotally mounted to the guide 705 (for example tubular body 701) and the respective engaging element 710, and the other being slidably mounted to the guide 705 (for example tubular body 701) and the respective engaging element. Compression of the scissor lift 745 in a longitudinal direction translates into movement of the engaging elements 710 in a lateral direction, to move to the expanded configurations. The device 100 may comprise one ratcheting actuators 746 slidably mounted to the guide 705 and configured to move the scissor lifts 745 between a plurality of positions corresponding to a plurality of expanded configurations of the expandable engaging elements 710. The ratcheting actuator 746 comprises a plurality of teeth (not shown) engaging with respective teeth on the guide 705 to allow movement in the distal direction D but not in the opposite direction. The device 700a may further comprise a release mechanism (not shown) for releasing the ratcheting mechanism (i.e. by disengaging the teeth) and allowing movement in the proximal direction opposite the distal direction D and therefore movement from the expanded configuration to the collapsed configuration.

In some embodiments, the guide 705 may comprise first branched guide 725 and second branched guide 730 at its proximal end defining respective lumens 707, 708 which branch from lumen 706. The branched guides 725 and 730 allow for separable components of the embryo transfer device (for example the measurement assembly and the inner body) to be inserted through the device 700a or 700b separately, whilst moving in unison when the device 700a or 700b is moved to position the embryo transfer device within the uterus.

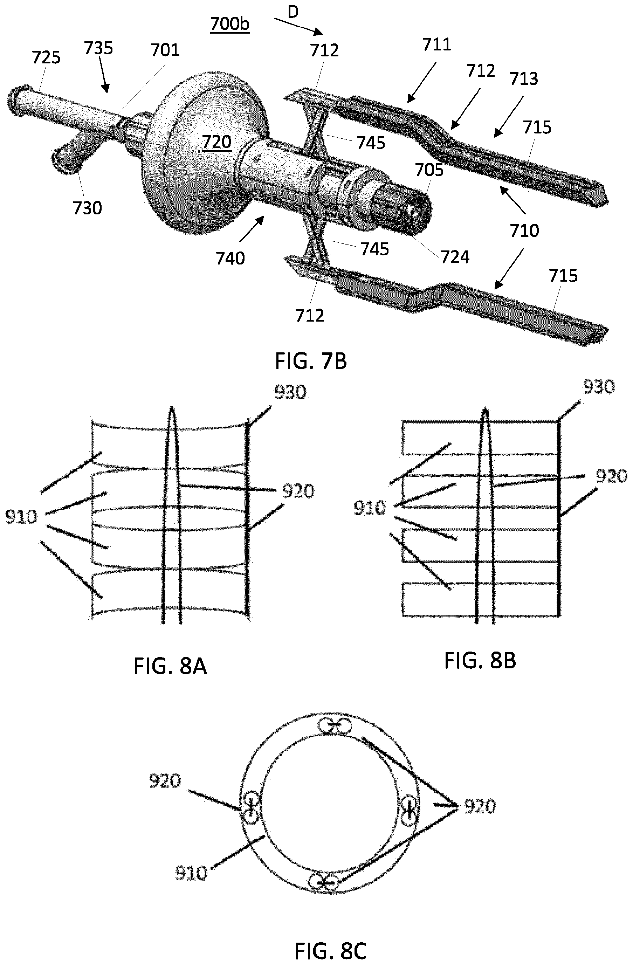

The expandable engaging elements 710 of Fig. 7A may be rectilinear as illustrated or may comprise any of the features of the engaging elements 710 disclosed with reference to Figs. 7B and 7C.

Fig. 7B shows a perspective view of another device 700b for engaging a speculum (also referred to as a speculum lock) in a collapsed configuration. Fig. 7C shows the device 700b of Fig. 7B in an expanded configuration. Again, the device 700b comprises two or more expandable engaging elements 710 for outwardly engaging a speculum to fix the device 700b to the speculum, wherein the two or more expandable engaging elements 710 comprise a plurality of expanded configurations for engaging a plurality of speculum sizes. The device further comprises a guide 705 for guiding an embryo delivery device between the expandable engaging elements 710 and through the speculum when the device 700b is fixed to the speculum. The guide 705 may comprise a tubular body 701 having a tubular wall defining a lumen for slidably receiving the embryo delivery device, wherein the cross-sectional shape of the inner surface of the tubular wall 702 at least partially conforms to the outer surface of the embryo delivery device to allow longitudinal movement of the embryo delivery device through the guide and to prevent lateral movement of the embryo delivery device in the guide.

The engaging elements 710 may be expandable using any suitable mechanism as described with reference to Fig. 7A. In the illustrated embodiment, the engaging elements 710 may be mounted to the guide 705 via respective scissor lifts 745. The scissor lifts 745 may comprise one or more pairs of struts pivotally connected to one another, with one strut being pivotally mounted to the guide 705 (for example tubular body 701) and the respective engaging element 710, and the other being slidably mounted to the guide 705 (for example tubular body 701) and the respective engaging element. The device 700b comprises a proximal part 735 and a distal part 740, which are slidably connected. In the illustrated embodiments, the proximal part 735 is slidably received within a lumen of the distal part 740. One strut of each scissor lift 745 is pivotally mounted to the proximal part 735 whilst the other is pivotally mounted to the distal part 740. When the proximal and distal parts 735, 740 are moved relative to one another, the scissor lifts are actuated so that the expandable engaging elements move between various expanded configurations. The device 700b also comprises a ratcheting actuator 750 comprising a plurality of teeth on the proximal part 735 engaging with respective teeth on the distal part 740 to allow relative movement of the proximal part 735 relative to the distal part 740 in one direction but not in the opposite direction. The device 700a may further comprise a release mechanism (not shown) for releasing the ratcheting mechanism (i.e. by disengaging the teeth) and allowing movement in the opposite of the proximal part 735 relative to the distal part 740 and therefore movement from the expanded configuration to the collapsed configuration. It will be appreciated that the direction of relative movement of the proximal part 735 and the distal part 740 which causes the engaging elements 710 to move to the expanded position will depend on which strut is pivotally attached to which part. In the illustrated embodiment, the struts are attached such that movement of the proximal part 735 in the distal direction D causes the engaging elements to move outwardly to move to the expanded configurations.

In some embodiments, one or more of the expandable engaging elements 710 comprise a strut 712 movable between a collapsed configuration (as shown in Fig. 7A) and a plurality of expanded configurations (as shown in Fig. 7B). The strut 712 may be rectilinear as illustrated in Fig. 7A or may have an outer portion 711, an inner portion 713 having a lower radial extent than the outer portion 711, and an intermediate portion 712 connecting the outer portion 711 and the inner portion 713. The struts of the scissor lift 745 are pivotally/slidably mounted to an inside surface of the outer portion 711. In any of the embodiments, the expandable engaging elements 710 may further comprise a frictional layer 715 mounted on the strut 712 and made of a material having a higher friction coefficient than the strut 712. The strut 712 provides structural support for the engaging element whilst the frictional layer provides a contact region for the speculum. The strut 712 may be made of for example, a metal and the frictional layer 715 may be made of any suitable material having a higher firction. The use of an outer portion 711 and an inner portion 713 allows the component parts of the device 700b external to the speculum to be less compact whilst still allowing a low radial profile at the distal end of the device 700b ready for insertion into the speculum, as can be seen in Fig. 7B.

The device 700b may comprise one or more branches at a proximal end. In the illustrated embodiment, the device 700b includes first and second branched guides 725 and 730 at its proximal end defining respective lumens which branch from the lumen of the tubular body 701.

The device 700b may also comprise a connecting mechanism 724 at the distal end of the guide 705, such as a Luer lock or a stab-fit connection, for connecting to a proximal end of another component of an embryo delivery apparatus such as the steering mechanism disclosed in Figs. 8A to 8H. The device 700b may further comprise a handle 720 fixed to the distal part 740.

In use, a speculum may be inserted into the vagina to dilate it. An embryo delivery device may be passed through the device 700a or 700b, through the speculum and past the cervix. The engaging elements 710 may be expanded to the expanded configuration so that the device 700a or 700b is fixed to the speculum. As a result, the embryo delivery device has restricted movement relative to the anatomy of the patient, allowing a higher controllability of the embryo delivery device.

Fig. 8A shows a side view of a steering mechanism configured to steer a distal portion of the body 110 according to some embodiments. The steering mechanism comprises a plurality of connected rings 910 forming a tube wall configured to at least partially surround a portion of the body 110. Specifically, the rings 910 are sized to receive the body 110 inside the tube wall, and may slidably receive the body 110. The mechanism further comprises at least one cable 920 extending longitudinally along the tube wall and attached to a distal end 930 of the tube wall. For example, the at least one cable may extend longitudinal along the inner or outer side of the tube wall or may extend longitudinally through the tube wall. The mechanism may comprise a plurality of said cables located at different circumferential positions about the tube wall. The connected rings 910 may be connected by the cables 920 alone. The connected rings 910 may further be configured to maintain a predetermined alignment by comprising a set of interlocking features which inhibit the rings 910 from rotating about the longitudinal axis. The steering mechanism shown in Fig. 8A comprises a plurality of connected rings 910 which are pivotable about each other. In other words, the connected rings are undulated such that neighbouring rings abut at a tangential contact point. This enables the rings 910 to roll about each other to steer the body 110 when one of the cables 920 are retracted by the third actuator (not shown). The circumferential position of the tangential contact points about which the ring 910 roll may be varied along the length of the steering mechanism. For example, sequential circumferential contact points may be offset by 90 degrees in order to accommodate rolling in two perpendicular directions by a plurality of cables. Preferably, the steering mechanism comprises two pairs of said cables, each pair comprising radially opposing cables, in order to provide steering in a plurality of directions. Such a configuration is shown in Fig. 8C, which is a top view of such a steering mechanism.

The connected rings shown in Fig. 8A may be made of any suitable material such as medical grade injection moldable or 3D printed plastic resin such as ABS, PET, DELRIN or PTFE. The cables 920 may be made of, for example, stainless steel and threaded through holes formed in the walls of the connected rings forming the tube wall. The cables 920 may each extend through a pair of holes in the connected rings and be looped at the distal end 930 as shown in Figs. 8A and 8C. The third actuator may be any suitable actuator for contracting the one or more cables.

Fig. 8B shows a side view of a steering mechanism according to an alternative embodiment. The steering mechanism comprises a plurality of connected rings 915 forming a tube wall configured to at least partially surround a portion of the body 110. Specifically, the rings 915 are sized to receive the body 110 inside the tube wall, and may slidably receive the body 110. The mechanism further comprises at least one cable 920 extending longitudinally through the tube wall and attached to a distal end 930 of the tube wall. The plurality of connected rings 910 are rings of a compressible tube or spring. When one of the cables is retracted by the third actuator (not shown), the tube or spring is compressed on the side of the retracted cable such that the steering mechanism is steered towards the contracted side. As the tube is compressible, the tension force is partly counteracted such that the resulting curve of the mechanism shown in 9B may be less than for the embodiment shown in Fig. 8A for a given tensioning force. Once again, preferably, the steering mechanism comprises two pairs of said cables, each pair comprising radially opposing cables, in order to provide steering in a plurality of directions, as shown in Fig. 8C.

The connected rings shown in Fig. 8B may be a compressible tube or spring made of any suitable material such as FEP, PTFE. The cables may be made of any suitable material such as stainless steel and threaded through holes formed in the walls of the connected rings forming the tube wall. The cables 920 may each extend through a pair of holes in the connected rings and be looped at the distal end 930 as shown in Figs. 8A and 8C. The third actuator may be any suitable actuator for contracting the one or more cables.

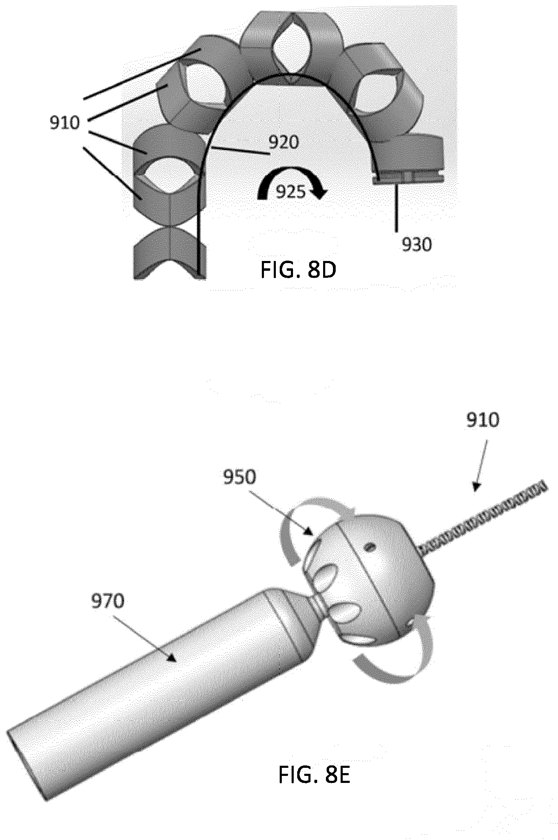

Fig. 8D shows a side view of a steering mechanism when one of the cables has been retracted, such that the steering mechanism is steered in the direction illustrated by arrow 925.

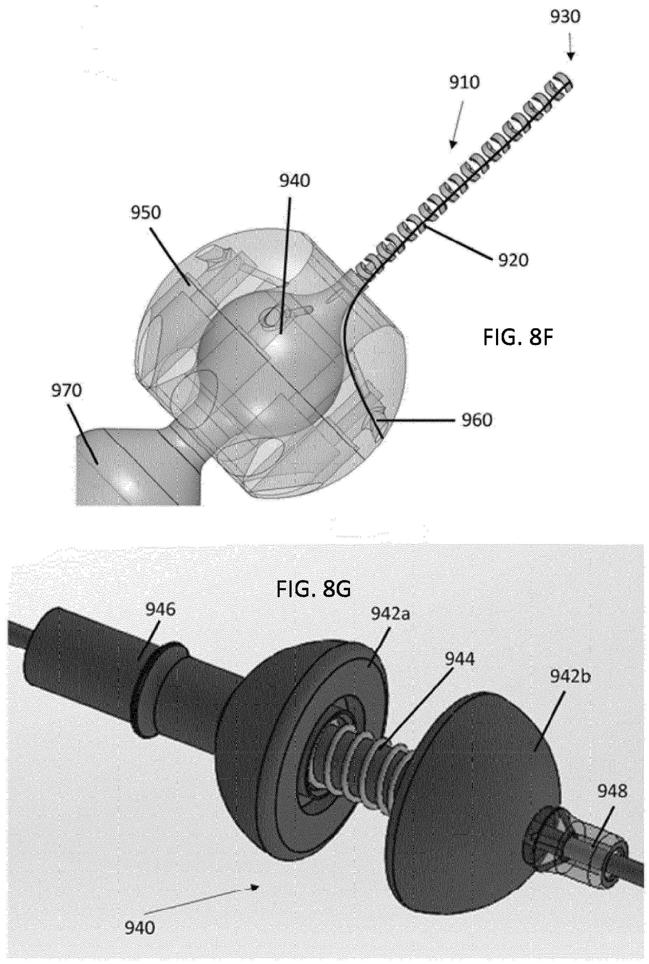

Figs. 8E shows a perspective view of the third actuator according to some embodiments. Fig. 8F shows a perspective view of the third actuator shown in Fig. 8E with the outer shell 950 depicted as transparent. The third actuator comprises an inner element 940 and an outer shell 950 housing the inner element 940. Each of the at least one cables 920 are fixed to the outer shell at a proximal portion 960, for example by welding, adhesive, or about a fixed winch. The inner element 940 is fixedly connected to the tube wall 910 either directly or indirectly via one or more intermediate components. The outer shell 950 is rotatable relative to the inner element 940. Optionally, a handle 970 may be fixed to the inner element 940 through the outer shell 950. Rotating the outer shell 950 about the inner element 940 causes one of the cables 920 to be retracted, which steers the tube wall in a certain direction as discussed above. The body may extend through the handle 970 and the inner element 940 such that it may be advanced through the steering mechanism.

Fig. 8G shows a perspective view of the inner element according to some embodiments. The inner element 940 may comprise one or more components which are biased to abut the outer shell 950. For example, the inner element 940 may comprise two hemispheres 942a, 942b, which are biased away from each other to abut the inner surface of the outer shell 950. The biasing causes a frictional fit to be obtained between the inner element 940 and outer shell 950, such that they may only slide relative to each other when a sufficient force is applied, meaning that once a desired direction of the steering mechanism is achieved, it may be maintained in position by the third actuator. The inner element may be connected to the handle via a handle connector 946, and may be connected to the tube wall via an intermediate portion 948. The intermediate portion 948 may be incompressible and the cables 920 may be threaded through the intermediate portion and to the tube wall.

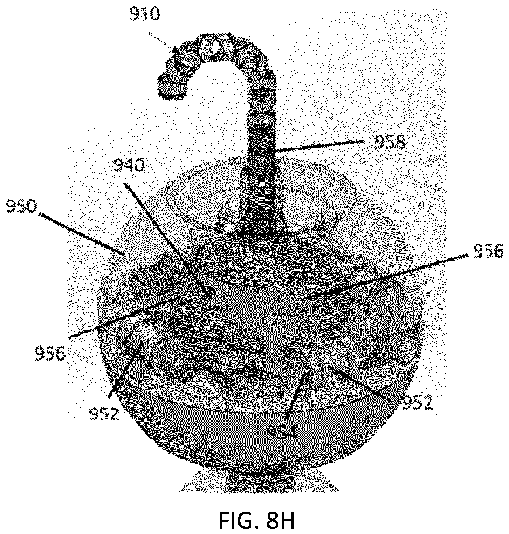

Fig. 8H shows a perspective view of a steering mechanism according to some embodiments, with the outer shell 950 illustrated as partially transparent. The steering mechanism of Fig. 8H also comprises an inner element 940 and an outer shell 950 housing the inner element 940 and rotatable relative to inner element 940, as described above. The steering mechanism may also comprise the handle 970. Further, each of the cables 920 are fixedly connected to the outer shell via a winch 952 (i.e. the cables are wound to the winch). The winch may be wound or unwound using an adjuster 954 (for example a flat head adjuster) which is accessible externally to the outer shell 950, meaning that the default tensions for each cable 920 may be calibrated after assembly of the steering mechanism. The rings 910 forming the tube wall may be fixedly connected to the inner element 940 via an inflexible tubular intermediate 958. The tubular intermediate 958 may be of any suitable length. For example, in embodiments where the outer catheter is used, the tubular intermediate 958 may be configured to extend through any inflexible parts of the outer catheter.

The cables 920 may extend through channels 956 formed in the inner surface of outer shell 950, and thus avoid contact with the inner element 940, avoiding damage to the cables as the outer shell rotates relative to the inner shell 940. The cables 920 may extend from the channels 956 and through lumens formed in the wall of the tubular intermediate 958, and to the tube wall.

The outer shell 950, inner element 940, handle 970, winches 952 and tubular intermediate 958 may be made of medical grade injection moldable or 3D printed plastic resin such as ABS, PET, DELRIN or PTFE.

The steering mechanism may instead be electronically controlled. For example, the steering mechanism shown in Fig. 8H may comprise an electronic controller fixed to the inner element 940, and comprising motors for actuating movement of the outer shell 950 relative to the inner element 940. Alternatively, a steering mechanism may comprise a plurality of connected rings forming a tube wall configured to at least partially surround a portion of the body, and at least two opposing cables extending longitudinally through the tube wall and attached to a distal end of the tube wall, wherein the cables are connected to a motor configured to contract the cables to steer the steering mechanism.

The tube wall of the steering mechanism is sized to slidably receive the body 110. The outer diameter of the tube wall may be about 1.4mm to 1.8mm or about 1.5mm to 1.7mm, for example 1.6mm. In embodiments where the steering mechanism is used, the body 110 may be configured to advance beyond the end of the steering mechanism by about 20mm to 40mm, for example about 30mm.

A proximal end of the steering mechanism may be configured to connect with a distal end of the speculum lock 700a or 700b. For example, the steering mechanism may comprise a connecting mechanism configured to connect with a corresponding connecting mechanism on the speculum lock 700a or 700b, so that the guide 705 and the steering mechanism define a continuous lumen through which body 110 slidably extends.

The following clauses also form part of the present disclosure:
1. A system for delivering a fertilized egg or embryo into a maternal uterine endometrium in humans or any other mammalian species, comprising:
   a body having a distal end suitable for penetrating the endometrial epithelium;
   a plunger slidably received in a lumen of the body;
   an actuator operable advance the plunger to expel a fertilized egg from the lumen of the body;
   a first injectable medium; and
   a second injectable medium for containing the fertilized egg;
   wherein the first injectable medium has a higher viscosity than the second injectable medium.
2. The system of clause 1, wherein the first injectable medium comprises or consists of one or more of the following: hyaluronic acid, a salt of hyaluronic acid, a biocompatible hydrogel, poloxamer, collagen, fibrinogen, gelatin, sodium hyaluronate and combinations thereof
3. The system of clause 1 or 2, wherein the first injectable medium and the second injectable medium are received in the lumen of the body, wherein the second injectable medium is located ahead of the first injectable medium relative to the distal end of the body such that the second injectable medium is expelled before the first injectable medium when the actuator is actuated to expel a fertilized egg.
4. The system of any preceding clause, further comprising a third injectable medium having a higher viscosity than the second medium, optionally wherein the third injectable medium comprises or consists of one or more of the following: hyaluronic acid, a salt of hyaluronic acid, a biocompatible hydrogel, poloxamer, collagen, fibrinogen, gelatin, sodium hyaluronate and combinations thereof.
5. The system of clause 4, wherein the first, second and third injectable medium are all received in the lumen of the body, wherein the second injectable medium is received between the first and third injectable mediums, such that the second medium is expelled before of one of the first and third injectable mediums, and after the other of the first and third injectable mediums, when the actuator is actuated to expel a fertilized egg.
6. The system of any preceding clause, wherein the actuator operable to advance the plunger is a first actuator and the body is an inner body, and the system further comprises:
   an outer body configured to fit within a lumen of the female reproductive system, the outer body comprising an outer lumen extending from a proximal end of the outer body to a distal portion of the outer body and having a distal opening at the distal portion of the outer body, the outer lumen configured to slidably receive the body; and
   a second actuator operable to advance the inner body out from the distal opening of the outer body.
7. A method of loading a system for delivering a fertilized egg or embryo into a maternal uterine endometrium in humans or any other mammalian species, the system comprising:
   a body having a distal end suitable for penetrating the endometrial epithelium;
   a plunger slidably received in a lumen of the body; and
   an actuator operable to advance the plunger to expel a fertilized egg from the lumen of
   the body;
   wherein the method comprises:
   drawing a first injectable medium into the lumen; and
   subsequently drawing a second injectable medium containing a fertilized egg into the lumen;
   wherein the first injectable medium has a higher viscosity than the second injectable medium.
8. The method of clause 7, wherein the first injectable medium comprises or consists of one or more of the following: hyaluronic acid, a salt of hyaluronic acid, a biocompatible hydrogel, poloxamer, collagen, fibrinogen, gelatin, sodium hyaluronate and combinations thereof.
9. The method of clause 7 or 9, further comprising subsequently drawing a third injectable medium into the inner lumen of the inner body after drawing the second injectable medium into the inner lumen of the inner body.
10. The method of clause 9, wherein the third injectable medium comprises or consists of one or more of the following: hyaluronic acid, a salt of hyaluronic acid, a biocompatible hydrogel, poloxamer, collagen, fibrinogen, gelatin, sodium hyaluronate and combinations thereof.
11. The method of any of clauses 7 to 10, wherein the actuator operable to advance the plunger is a first actuator and the body is an inner body, and the system further comprises:
   an outer body configured to fit within a lumen of the female reproductive system, the body comprising an outer lumen extending from a proximal end of the outer body to a distal portion of the outer body and having a distal opening at the distal portion of the outer body, the outer lumen configured to slidably receive the body; and
   a second actuator operable to advance the inner body out from the distal opening of the outer body.
12. An apparatus suitable for delivering a fertilized egg or embryo into a maternal uterine endometrium in humans or any other mammalian species, comprising a body configured to fit within a lumen of the female reproductive system, the body comprising:
   one or more lumens extending from a proximal end of the body to a distal portion of the body and having a distal opening at the distal portion of the body, wherein a first lumen of the one or more lumens slidably receives an inner body having a distal end suitable for penetrating the endometrial epithelium, the inner body comprising an inner lumen for receiving a fertilized egg;
   an actuator operable to expel the fertilized egg from the inner lumen; and
   a covering layer at least partially covering the distal opening of at least one lumen of the one or more lumens.
13. The apparatus of clause 12, wherein the covering layer at least partially covers the distal opening of the first lumen and the inner body is configured to perforate though the covering layer when the inner body is advanced out from the distal opening of the body.
14. The apparatus of clause 12 or 13, further comprising a measurement assembly, the measurement assembly extending through a lumen of the one or more lumens and comprising a measurement portion proximal a distal opening of the one or more lumens, the measurement portion configured to measure whether the apparatus is in a first state indicating that a distance between the distal end of the body or the inner body and the endometrial epithelium is greater than a predetermined distance, or a second state indicating that a distance between the distal end of the body or the inner body and the endometrial epithelium is equal to or less than a predetermined distance; wherein the measurement portion is at least partially covered by the covering layer.
15. The apparatus of clause 14, wherein the measurement assembly comprises a capacitance sensor configured to make electrical contact with a distal portion of the inner body which is electrically conductive, and to provide an indication of capacitance, wherein the first state is indicated by a capacitance measurement below a threshold value and the second state is indicated by a capacitance measurement above the threshold value.
16. The apparatus of any of clauses 12 to 15, further comprising a camera proximal the distal opening and configured to view a portion of the endometrial epithelium.
17. The apparatus of clause 16, wherein the covering layer covers the camera so that the camera is not exposed to mucus when the distal portion of the apparatus is placed in the lumen of the female reproductive system.
18. The apparatus of any of clauses 12 to 17, wherein the covering layer comprises a biocompatible transparent or semi-transparent film, preferably wherein the covering layer is a transparent or semi-transparent film comprising or consisting of any of the following: polyolefin, PVC, paraffin, cellulose and derivates, hyaluronic acid thin films, parylene C-films, collagen, gelatin, vegetable casings, alginates and composites or combinations thereof.
19. A device for engaging a speculum, comprising:
   two or more expandable engaging elements for outwardly engaging a speculum to fix the device to the speculum, wherein the two or more expandable engaging elements comprise a plurality of expanded configurations for engaging a plurality of speculum sizes; and
   a guide for guiding an embryo delivery device between the expandable engaging elements and through the speculum when the device is fixed to the speculum.
20. The device of clause 19, further comprising the embryo delivery device, wherein the guide at least partially conforms to an outer surface of the embryo delivery device to allow longitudinal movement of the embryo delivery device through the guide and to prevent lateral movement of the embryo delivery device in the guide.
21. The device of clause 19 or 20, wherein the engaging elements are mounted to the guide by respective scissor lifts, the device further comprising a ratcheting actuator allowing movement of the scissor lifts in an outer direction to a plurality of expanded configurations of the expandable engaging elements, and a release mechanism for disengaging the ratcheting actuator.
22. The device of any of clauses 19 to 21, wherein the guide comprises intersecting first and second branched guides at its proximal end which intersect to form a single guide extending towards the distal end.
23. The device of any of clauses 19 to 22, wherein one or more of the expandable engaging elements comprise an outer portion, and inner portion having a lower radial extent than the outer portion, and an intermediate portion connecting the outer portion and the inner portion.
24. The device of any of clauses 19 to 23, wherein one or more of the expandable engaging elements comprise a strut and a frictional layer mounted on the strut and made of a material having a higher friction coefficient than the strut.
25. An apparatus suitable for delivering a fertilized egg or embryo into a maternal uterine endometrium in humans or any other mammalian species, comprising a body configured to fit within a lumen of the female reproductive system, the body comprising:
   a lumen extending from a proximal end of the body to a distal portion of the body and having a distal opening at the distal portion of the body, the lumen slidably receiving an inner body having a distal end suitable for penetrating the endometrial epithelium;
   the apparatus further comprising:
   a first actuator operable to advance the inner body out from the distal opening of the body; and
   a second actuator operable to expel a fertilized egg from an inner lumen of the inner body;
   wherein the inner body comprises a hydrophobic coating formed on a distal portion of the inner body.
26. The apparatus of clause 25, wherein the hydrophobic coating is provided on at least a distal portion of an inner surface of the inner body defining the inner lumen.
27. The apparatus of clause 25 or 26, wherein the hydrophobic coating is provided on an outer surface and/or the distal tip of the inner body.
28. The apparatus of any of clauses 25 to 27, wherein the inner body comprises a bevelled tip.
29. The apparatus of any of clauses 25 to 28, wherein the hydrophobic coating comprises or consists of a polymer or polymer composite, preferably any of the following: parylene, acrylic, polyethylene, polyurethane and polytetrafluorethylene and composites thereof.

All of the above are fully within the scope of the present disclosure, and are considered to form the basis for alternative embodiments in which one or more combinations of the above described features are applied, without limitation to the specific combination disclosed above.

In light of this, there will be many alternatives which implement the teaching of the present disclosure. It is expected that one skilled in the art will be able to modify and adapt the above disclosure to suit its own circumstances and requirements within the scope of the present disclosure, while retaining some or all technical effects of the same, either disclosed or derivable from the above, in light of his common general knowledge in this art. All such equivalents, modifications or adaptations fall within the scope of the present disclosure.

## Claims

1. A system for delivering a fertilized egg or embryo into a maternal uterine endometrium in humans or any other mammalian species, comprising:
a body having a distal end suitable for penetrating the endometrial epithelium;
a plunger slidably received in a lumen of the body;
an actuator operable advance the plunger to expel a fertilized egg from the lumen of the body;
a first injectable medium; and
a second injectable medium for containing the fertilized egg;
wherein the first injectable medium has a higher viscosity than the second injectable medium.

2. The system of claim 1, wherein the first injectable medium comprises or consists of one or more of the following: hyaluronic acid, a salt of hyaluronic acid, a biocompatible hydrogel, poloxamer, collagen, fibrinogen, gelatin, sodium hyaluronate and combinations thereof

3. The system of claim 1 or 2, wherein the first injectable medium and the second injectable medium are received in the lumen of the body, wherein the second injectable medium is located ahead of the first injectable medium relative to the distal end of the body such that the second injectable medium is expelled before the first injectable medium when the actuator is actuated to expel a fertilized egg.

4. The system of any preceding claim, further comprising a third injectable medium having a higher viscosity than the second medium, optionally wherein the third injectable medium comprises or consists of one or more of the following: hyaluronic acid, a salt of hyaluronic acid, a biocompatible hydrogel, poloxamer, collagen, fibrinogen, gelatin, sodium hyaluronate and combinations thereof.

5. The system of claim 4, wherein the first, second and third injectable medium are all received in the lumen of the body, wherein the second injectable medium is received between the first and third injectable mediums, such that the second medium is expelled before of one of the first and third injectable mediums, and after the other of the first and third injectable mediums, when the actuator is actuated to expel a fertilized egg.

6. The system of any preceding claim, wherein the actuator operable to advance the plunger is a first actuator and the body is an inner body, and the system further comprises:
an outer body configured to fit within a lumen of the female reproductive system, the outer body comprising an outer lumen extending from a proximal end of the outer body to a distal portion of the outer body and having a distal opening at the distal portion of the outer body, the outer lumen configured to slidably receive the body; and
a second actuator operable to advance the inner body out from the distal opening of the outer body.

7. A method of loading a system for delivering a fertilized egg or embryo into a maternal uterine endometrium in humans or any other mammalian species, the system comprising:
a body having a distal end suitable for penetrating the endometrial epithelium;
a plunger slidably received in a lumen of the body; and
an actuator operable to advance the plunger to expel a fertilized egg from the lumen of the body;
wherein the method comprises:
drawing a first injectable medium into the lumen; and
subsequently drawing a second injectable medium containing a fertilized egg into the lumen;
wherein the first injectable medium has a higher viscosity than the second injectable medium.

8. The method of claim 7, wherein the first injectable medium comprises or consists of one or more of the following: hyaluronic acid, a salt of hyaluronic acid, a biocompatible hydrogel, poloxamer, collagen, fibrinogen, gelatin, sodium hyaluronate and combinations thereof.

9. The method of claim 7 or 9, further comprising subsequently drawing a third injectable medium into the inner lumen of the inner body after drawing the second injectable medium into the inner lumen of the inner body.

10. The method of claim 9, wherein the third injectable medium comprises or consists of one or more of the following: hyaluronic acid, a salt of hyaluronic acid, a biocompatible hydrogel, poloxamer, collagen, fibrinogen, gelatin, sodium hyaluronate and combinations thereof.

11. The method of any of claims 7 to 10, wherein the actuator operable to advance the plunger is a first actuator and the body is an inner body, and the system further comprises:
an outer body configured to fit within a lumen of the female reproductive system, the body comprising an outer lumen extending from a proximal end of the outer body to a distal portion of the outer body and having a distal opening at the distal portion of the outer body, the outer lumen configured to slidably receive the body; and
a second actuator operable to advance the inner body out from the distal opening of the outer body.

12. An apparatus suitable for delivering a fertilized egg or embryo into a maternal uterine endometrium in humans or any other mammalian species, comprising a body configured to fit within a lumen of the female reproductive system, the body comprising:
one or more lumens extending from a proximal end of the body to a distal portion of the body and having a distal opening at the distal portion of the body, wherein a first lumen of the one or more lumens slidably receives an inner body having a distal end suitable for penetrating the endometrial epithelium, the inner body comprising an inner lumen for receiving a fertilized egg;
an actuator operable to expel the fertilized egg from the inner lumen; and
a covering layer at least partially covering the distal opening of at least one lumen of the one or more lumens.

13. The apparatus of claim 12, wherein the covering layer at least partially covers the distal opening of the first lumen and the inner body is configured to perforate though the covering layer when the inner body is advanced out from the distal opening of the body.

14. The apparatus of claim 12 or 13, further comprising a measurement assembly, the measurement assembly extending through a lumen of the one or more lumens and comprising a measurement portion proximal a distal opening of the one or more lumens, the measurement portion configured to measure whether the apparatus is in a first state indicating that a distance between the distal end of the body or the inner body and the endometrial epithelium is greater than a predetermined distance, or a second state indicating that a distance between the distal end of the body or the inner body and the endometrial epithelium is equal to or less than a predetermined distance; wherein the measurement portion is at least partially covered by the covering layer.

15. The apparatus of claim 14, wherein the measurement assembly comprises a capacitance sensor configured to make electrical contact with a distal portion of the inner body which is electrically conductive, and to provide an indication of capacitance, wherein the first state is indicated by a capacitance measurement below a threshold value and the second state is indicated by a capacitance measurement above the threshold value.

16. The apparatus of any of claims 12 to 15, further comprising a camera proximal the distal opening and configured to view a portion of the endometrial epithelium.

17. The apparatus of claim 16, wherein the covering layer covers the camera so that the camera is not exposed to mucus when the distal portion of the apparatus is placed in the lumen of the female reproductive system.

18. The apparatus of any of claims 12 to 17, wherein the covering layer comprises a biocompatible transparent or semi-transparent film, preferably wherein the covering layer is a transparent or semi-transparent film comprising or consisting of any of the following: polyolefin, PVC, paraffin, cellulose and derivates, hyaluronic acid thin films, parylene C-films, collagen, gelatin, vegetable casings, alginates and composites thereof.

19. A device for engaging a speculum, comprising:
two or more expandable engaging elements for outwardly engaging a speculum to fix the device to the speculum, wherein the two or more expandable engaging elements comprise a plurality of expanded configurations for engaging a plurality of speculum sizes; and
a guide for guiding an embryo delivery device between the expandable engaging elements and through the speculum when the device is fixed to the speculum.

20. The device of claim 19, further comprising the embryo delivery device, wherein the guide at least partially conforms to an outer surface of the embryo delivery device to allow longitudinal movement of the embryo delivery device through the guide and to prevent lateral movement of the embryo delivery device in the guide.

21. The device of claim 19 or 20, wherein the engaging elements are mounted to the guide by respective scissor lifts, the device further comprising a ratcheting actuator allowing movement of the scissor lifts in an outer direction to a plurality of expanded configurations of the expandable engaging elements, and a release mechanism for disengaging the ratcheting actuator.

22. The device of any of claims 19 to 21, wherein the guide comprises intersecting first and second branched guides at its proximal end which intersect to form a single guide extending towards the distal end.

23. The device of any of claims 19 to 22, wherein one or more of the expandable engaging elements comprise an outer portion, and inner portion having a lower radial extent than the outer portion, and an intermediate portion connecting the outer portion and the inner portion.

24. The device of any of claims 19 to 23, wherein one or more of the expandable engaging elements comprise a strut and a frictional layer mounted on the strut and made of a material having a higher friction coefficient than the strut.

25. An apparatus suitable for delivering a fertilized egg or embryo into a maternal uterine endometrium in humans or any other mammalian species, comprising a body configured to fit within a lumen of the female reproductive system, the body comprising:
a lumen extending from a proximal end of the body to a distal portion of the body and having a distal opening at the distal portion of the body, the lumen slidably receiving an inner body having a distal end suitable for penetrating the endometrial epithelium;
the apparatus further comprising:
a first actuator operable to advance the inner body out from the distal opening of the body; and
a second actuator operable to expel a fertilized egg from an inner lumen of the inner body;
wherein the inner body comprises a hydrophobic coating formed on a distal portion of the inner body.

26. The apparatus of claim 25, wherein the hydrophobic coating is provided on at least a distal portion of an inner surface of the inner body defining the inner lumen.

27. The apparatus of claim 25 or 26, wherein the hydrophobic coating is provided on an outer surface and/or the distal tip of the inner body.

28. The apparatus of any of claims 25 to 27, wherein the inner body comprises a bevelled tip.

29. The apparatus of any of claims 25 to 28, wherein the hydrophobic coating comprises or consists of a polymer or polymer composite, preferably any of the following: parylene, acrylic, polyethylene, polyurethane and polytetrafluorethylene and composites thereof.
